# EUROPEAN PATENT APPLICATION

(11) **EP 4 471 035 A1**
(43) Date of publication of application: **04.12.2024**
(21) Application number: 23746090.2
(22) Date of filing: 16.01.2023
(51) Int. Cl.: C07D 491/00, C07D 491/22, C07K 5/062

(54) **PREPARATION METHOD FOR DRUG LINKER CONJUGATE**

(30) Priority: 26.01.2022 CN 202210095771
(71) Applicant: Medilink Therapeutics (Suzhou) Co., Ltd., Suzhou, Jiangsu 215000 (CN)
(72) Inventor: SONG, Shuai, Suzhou, Jiangsu 215000 (CN); LI, Jundong, Suzhou, Jiangsu 215000 (CN); YAN, Bin, Suzhou, Jiangsu 215000 (CN); ZHANG, Xiaozhou, Suzhou, Jiangsu 215000 (CN); WANG, Zizhen, Suzhou, Jiangsu 215000 (CN); CAI, Jiaqiang, Suzhou, Jiangsu 215000 (CN)
(74) Representative: Greaves Brewster LLP
(86) International application number: PCT/CN2023/072443
(87) International publication number: WO 2023/143208

(57) **Abstract**

The present invention provides a preparation method for a drug linker conjugate. The present invention further provides a preparation method for an intermediate or a salt thereof, and a use of an intermediate or a salt thereof. The raw materials involved in the preparation method of the present invention are easily obtained, the operation is simple, the purity of a product is high, and the present invention is suitable for high-volume synthesis.

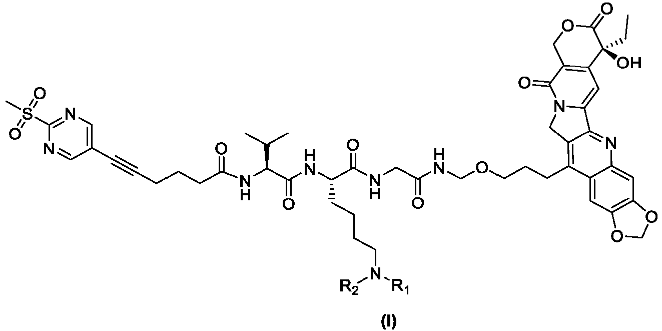

## Description

The present application claims the right of the priority of Chinese patent application 2022100957718 filed on January 26, 2022. The contents of the above Chinese patent application are incorporated herein by reference in their entireties.

### TECHNICAL FIELD

The present application belongs to the field of medicinal chemistry, and specifically relates to a preparation method for a drug linker conjugate or a salt thereof, and a related intermediate or a salt thereof.

### BACKGROUND

Antibody-drug conjugate (ADC) is a conjugate of antibody and small molecule drug, which combines the tumor targeting effect of antibodies with the activity of bioactive molecules. ADC has become a biological missile with promising advantage of efficacy and safety. The antibody guides the ADC to bind to target cells/tissues, and then small molecule drugs are released inside the cells/tissues by enzymatic hydrolysis under the action of specific enzymes to treat diseases. Among them, drug linker conjugates (including cytotoxic payload and linker) play an extremely important role in the preparation of ADCs. Therefore, the development of drug linker conjugate synthesis methods that are simple, efficient, low-cost, and suitable for large-scale synthesis is of great significance for the development and application of ADC drugs.

### CONTENT OF THE PRESENT INVENTION

The present application provides a preparation method for a drug linker conjugate (compound of formula I), a preparation method for an intermediate used or a salt thereof, and a use of the intermediate or the salt thereof. The preparation method of the present application involves readily available raw materials, simple operation, high product purity, and is suitable for large-scale synthesis.

In one aspect, the present application provides a preparation method for a compound of formula I or a salt thereof, 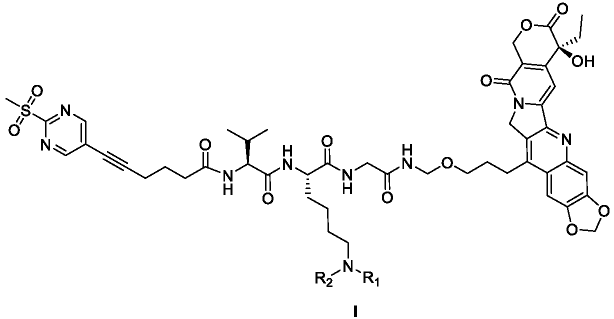 comprising the following steps:
c) reacting a compound of formula 6 or a salt thereof with a compound of formula 7 or a salt thereof to obtain a compound of formula 8 or a salt thereof, 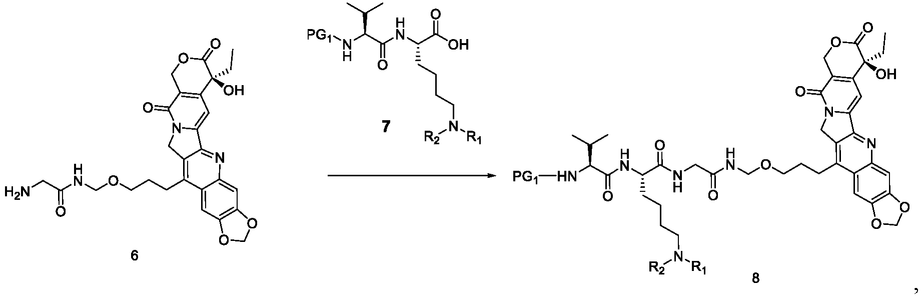
d) reacting the compound of formula 8 or the salt thereof to obtain a compound of formula 9 or a salt thereof, 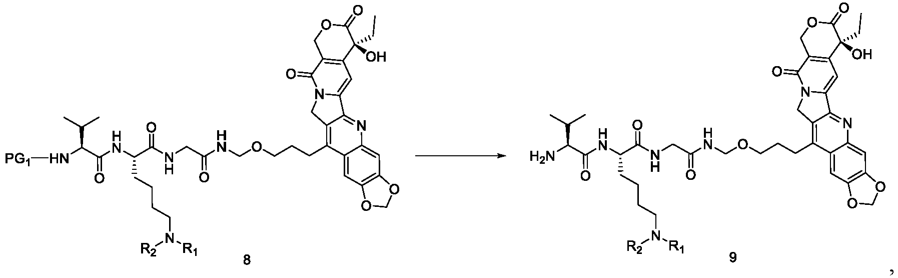
e) reacting the compound of formula 9 or the salt thereof with a compound of formula 10 to obtain the compound of formula I or the salt thereof, 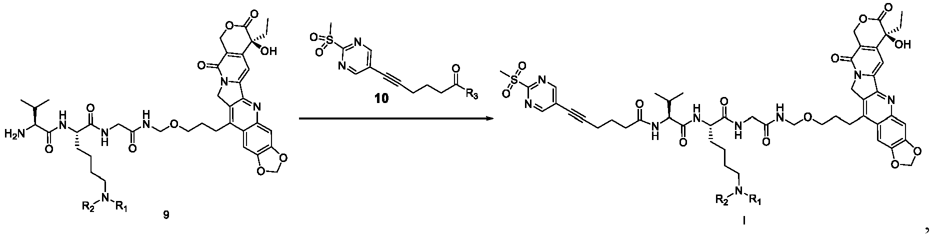 wherein
   R₁ and R₂ are each independently selected from C₁₋₄ alkyl;
   R₃ is selected from hydroxyl and halogen;
   PG₁ is selected from an amino protecting group.

In some embodiments, the preparation method for the compound of formula I or the salt thereof further comprises the following steps:
a) reacting a compound of formula 3 or a salt thereof with a compound of formula 4 to obtain a compound of formula 5 or a salt thereof, 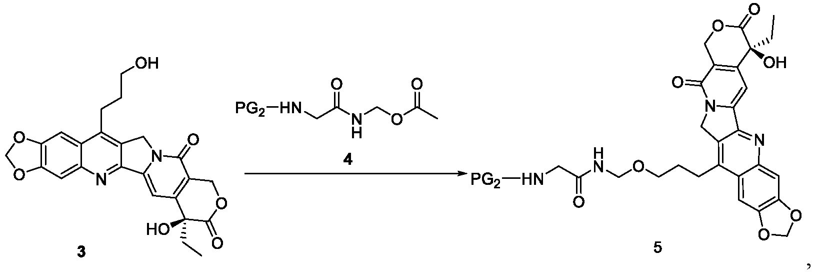
b) reacting the compound of formula 5 or the salt thereof to obtain the compound of formula 6 or the salt thereof, 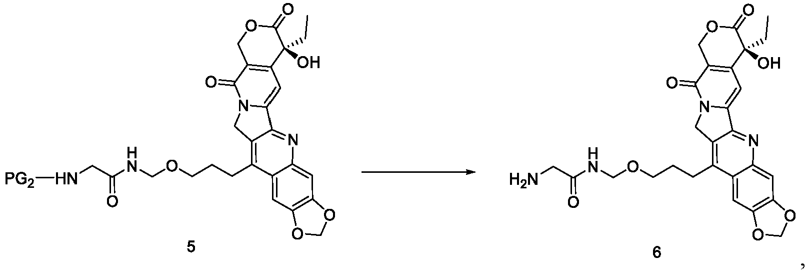
wherein PG₂ is selected from an amino protecting group.

In another aspect, the present application provides a preparation method for the compound of formula 7 or the salt thereof, comprising the following steps:
f-1) reacting a compound of formula 7-A1 with a compound of formula 7-A2 or a salt thereof to obtain a compound of formula 7-A3, 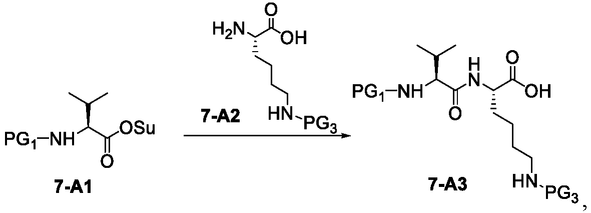
f-2) reacting the compound of formula 7-A3 to obtain a compound of formula 7-A4 or a salt thereof, 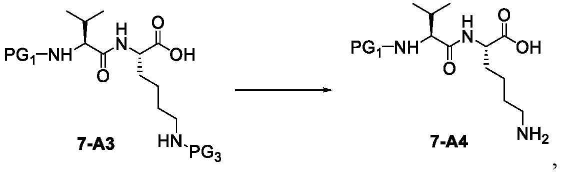
f-3) reacting the compound of formula 7-A4 or the salt thereof to obtain the compound of formula 7 or the salt thereof, 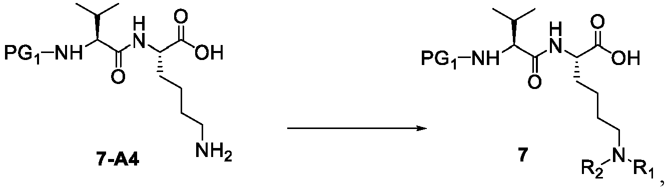 wherein
   R₁ and R₂ are each independently selected from C₁₋₄ alkyl;
   PG₁ and PG₃ are each independently selected from an amino protecting group, and PG₁ and PG₃ are different.

In another aspect, the present application provides a preparation method for the compound of formula **7** or the salt thereof, comprising the following steps:
g-1) reacting a compound of formula 7-B 1 or a salt thereof to obtain a compound of formula 7-B2 or a salt thereof, 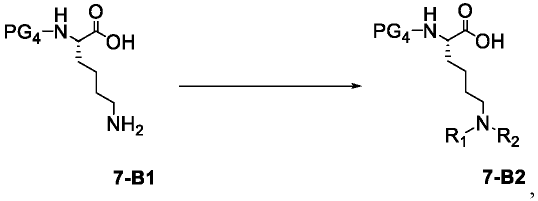
g-2) reacting the compound of formula 7-B2 or the salt thereof to obtain a compound of formula 7-B3 or a salt thereof, 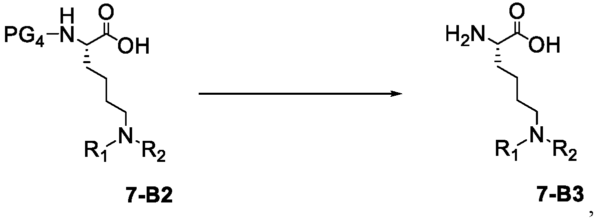
g-3) reacting the compound of formula 7-B3 or the salt thereof with the compound of formula 7-A1 to obtain the compound of formula 7 or the salt thereof, 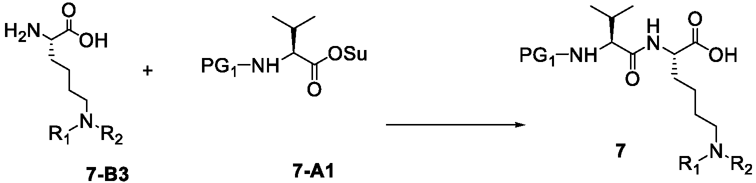 wherein
   R₁ and R₂ are each independently selected from C₁₋₄ alkyl;
   PG₁ and PG₄ are each independently selected from an amino protecting group.

In another aspect, the present application provides a preparation method for the compound of formula **3** or the salt thereof, comprising the following steps:
h) reacting a compound of formula 1 or a salt thereof with a compound of formula 2 to obtain the compound of formula 3 or the salt thereof, 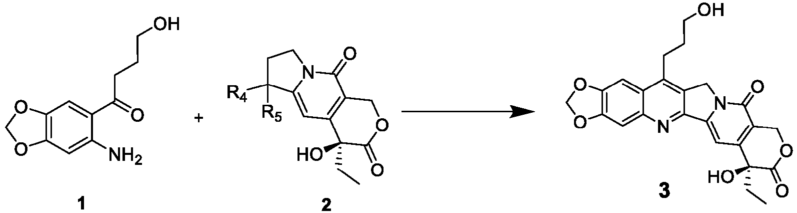
wherein
R₄ and R₅ are each independently selected from -OC₁₋₄ alkyl, -SC₁₋₄ alkyl, and -NHC₁₋₄ alkyl;
or, R₄ and R₅ together with the carbon atom to which they are attached form a carbonyl group;
or, R₄ and R₅ together with the carbon atom to which they are attached form 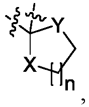 wherein X and Y are each independently selected from O, S, NH, and NC₁₋₄ alkyl, and n is selected from 1, 2, and 3.

In another aspect, the present application provides a preparation method for the compound of formula **1,** comprising the following steps:
step i-1) reacting a compound of formula 1-A1 with a compound of formula 1-A2 to obtain a compound of formula 1-A3;
step i-2) reacting the compound of formula 1-A3 to obtain the compound of formula 1 or the salt thereof;

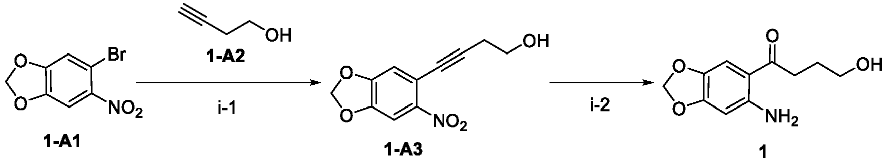

In another aspect, the present application provides a preparation method for the compound of formula **1** or the salt thereof, comprising the following steps:
step j-1) reacting a compound of formula 1-B1 with a compound of formula 1-B2 to obtain a compound of formula 1-B3;
step j-2) reacting the compound of formula 1-B3 to obtain a compound of formula 1-B4;
step j-3) reacting the compound of formula 1-B4 to obtain a compound of formula 1-B5 or a salt thereof;
step j-4) reacting the compound of formula 1-B5 or the salt thereof to obtain the compound of formula 1 or the salt thereof; 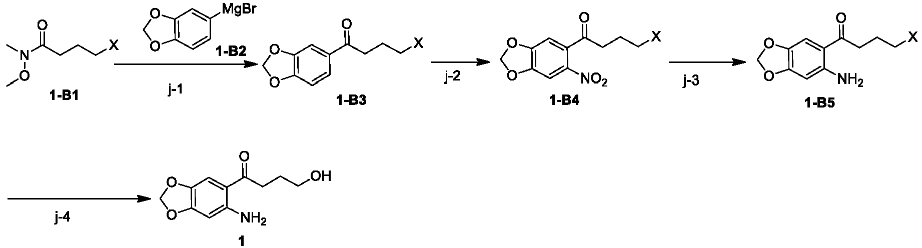
wherein X is selected from halogen.

In some embodiments, the R₁ and R₂ are each independently selected from methyl, ethyl, and propyl, preferably R₁ and R₂ are the same.

In some embodiments, both R₁ and R₂ are propyl.

In some embodiments, the R₃ is selected from hydroxyl, fluorine, chlorine, bromine, and iodine.

In some embodiments, the R₃ is selected from hydroxyl and chlorine.

In some embodiments, the X is selected from fluorine, chlorine, bromine, and iodine.

In some embodiments, the X is chlorine.

In some embodiments, the PG₁ is selected from an alkoxycarbonyl protecting group; preferably benzyloxycarbonyl (Cbz), *tert*-butoxycarbonyl (Boc), 9-fluorenylmethoxycarbonyl (Fmoc), allyloxycarbonyl (Alloc), (trimethylsilyl)ethoxycarbonyl (Teoc), methoxycarbonyl, or ethoxycarbonyl; more preferably 9-fluorenylmethoxycarbonyl (Fmoc).

In some embodiments, the PG₂ is selected from an alkoxycarbonyl protecting group; preferably benzyloxycarbonyl (Cbz), *tert*-butoxycarbonyl (Boc), 9-fluorenylmethoxycarbonyl (Fmoc), allyloxycarbonyl (Alloc), (trimethylsilyl)ethoxycarbonyl (Teoc), methoxycarbonyl, or ethoxycarbonyl; more preferably 9-fluorenylmethoxycarbonyl (Fmoc).

In some embodiments, the PG₃ is selected from an alkoxycarbonyl protecting group; preferably benzyloxycarbonyl (Cbz), *tert*-butoxycarbonyl (Boc), 9-fluorenylmethoxycarbonyl (Fmoc), allyloxycarbonyl (Alloc), (trimethylsilyl)ethoxycarbonyl (Teoc), methoxycarbonyl, or ethoxycarbonyl; more preferably *tert*-butoxycarbonyl (Boc).

In some embodiments, the PG₄ is selected from an alkoxycarbonyl protecting group; preferably benzyloxycarbonyl (Cbz), *tert*-butoxycarbonyl (Boc), 9-fluorenylmethoxycarbonyl (Fmoc), allyloxycarbonyl (Alloc), (trimethylsilyl)ethoxycarbonyl (Teoc), methoxycarbonyl, or ethoxycarbonyl; more preferably 9-fluorenylmethoxycarbonyl (Fmoc).

In some embodiments, the R₄ and R₅ are each independently selected from methoxy, ethoxy, propoxy, methylthio, ethylthio, propylthio, methylamino, ethylamino, and propylamino;
or, R₄ and R₅ together with the carbon atom to which they are attached form a carbonyl group;
or, R₄ and R₅ together with the carbon atom to which they are attached form 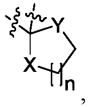 wherein X and Y are each independently selected from O, S, NH, and NC₁₋₄ alkyl, and n is selected from 1, 2, and 3.

In some embodiments, the R₄ and R₅ together with the carbon atom to which they are attached form a carbonyl group; or, R₄ and R₅ together with the carbon atom to which they are attached form

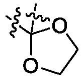

In some embodiments, in step a), the reaction is carried out in the presence of an acid. The acid is selected from hydrogen chloride, hydrobromic acid, sulfuric acid, formic acid, acetic acid, trifluoroacetic acid, p-toluenesulfonic acid, pyridinium *p*-toluenesulfonate, zinc acetate, aluminum trichloride (AlCl₃), ferric chloride (FeCl₃), and boron trifluoride diethyl etherate (BF₃·Et₂O); for example, hydrogen chloride or boron trifluoride diethyl etherate; preferably hydrogen chloride, *p*-toluenesulfonic acid, or zinc acetate.

In some embodiments, in step a), the molar ratio of the compound of formula 3 or the salt thereof to the acid selected for the reaction is selected from 1:0.2 to 1:2.

In some embodiments, in step a), the reaction is carried out in the presence of a base. The base is selected from sodium hydroxide, potassium *tert*-butoxide, potassium carbonate, triethylamine (Et₃N), *N,N*-diisopropylethylamine (DIPEA), and pyridine; preferably potassium *tert*-butoxide or sodium hydroxide.

In some embodiments, in step a), the molar ratio of the compound of formula 3 or the salt thereof to the base selected for the reaction is selected from 1:0.5 to 1:3.

In some embodiments, in step a), the molar ratio of the compound of formula 3 or the salt thereof to the compound of formula 4 is selected from 1:1 to 1: 10; preferably 1:2 to 1:6.

In some embodiments, in step a), reaction solvent is selected from an aprotic solvent; preferably an ether, haloalkane, ketone, nitrile, amide, or sulfone solvent; more preferably diethyl ether, diisopropyl ether, methyl *tert*-butyl ether, ethylene glycol diethyl ether, tetrahydrofuran, 1,4-dioxane, dichloromethane, 1,2-dichloroethane, acetone, *N,N-*dimethylformamide, *N,N*-dimethylacetamide, or dimethyl sulfoxide; further preferably diisopropyl ether, tetrahydrofuran, 1,4-dioxane, or *N,N*-dimethylformamide.

In some embodiments, in step a), the mass/volume ratio (g/mL) of the compound of formula 3 or the salt thereof to the selected solvent is selected from 1:5 to 1:50; preferably 1:8 to 1:30.

In some embodiments, in step a), reaction temperature is selected from -10 to 110°C; preferably 0 to 60°C.

In some embodiments, in step b), the protecting group PG₂ on the amino group is removed from the compound of formula 5 or the salt thereof to obtain the compound of formula 6 or the salt thereof.

In some embodiments, in step b), the reaction in which the protecting group PG₂ on the amino group is removed from the compound of formula 5 or the salt thereof can be carried out under conventional reaction conditions well known to those skilled in the art, for example, removal in the presence of an acid; removal in the presence of a base; removal under the catalysis of a palladium catalyst.

In some embodiments, in step b), when the PG₂ is 9-fluorenylmethoxycarbonyl (Fmoc), the protecting group PG₂ is removed from the compound of formula 5 or the salt thereof in the presence of a base. The base is selected from piperidine, diethylamine, morpholine, diisopropylamine, DBU, triethylamine, and *N,N*-diisopropylethylamine; preferably piperidine, diethylamine, or morpholine.

In some embodiments, in step b), when the PG₂ is 9-fluorenylmethoxycarbonyl (Fmoc), the molar ratio of the compound of formula 5 or the salt thereof to the selected base is selected from 1:0.2 to 1:40; preferably 1:1 to 1:10.

In some embodiments, in step b), when the PG₂ is 9-fluorenylmethoxycarbonyl (Fmoc), the reaction solvent is selected from one of DMF, DMAc, dichloromethane, tetrahydrofuran, 1,4-dioxane, acetonitrile, methanol, and ethanol, or any combination thereof; preferably one of DMF and dichloromethane, or any combination thereof.

In some embodiments, in step b), when the PG₂ is 9-fluorenylmethoxycarbonyl (Fmoc), the mass/volume ratio (g/mL) of the compound of formula 5 or the salt thereof to the selected reaction solvent is selected from 1:5 to 1:50; preferably 1:10 to 1:30.

In some embodiments, in step b), when the PG₂ is 9-fluorenylmethoxycarbonyl (Fmoc), the reaction temperature is selected from 0 to 50°C; preferably 10 to 30°C.

In some embodiments, in step c), the compound of formula 6 or the salt thereof is reacted with the compound of formula 7 or the salt thereof in the presence of a condensing agent to obtain the compound of formula 8 or the salt thereof. The condensing agent is selected from DMTMM, HATU, HBTU, HOBt/EDCI, and T₃P; preferably DMTMM or HOBt/EDCI.

In some embodiments, in step c), the molar ratio of the compound of formula 6 or the salt thereof to the condensing agent is selected from 1:0.7 to 1:5; preferably 1:0.7 to 1:2.

In some embodiments, in step c), the molar ratio of the compound of formula 6 or the salt thereof to the compound of formula 7 or the salt thereof is selected from 1:0.7 to 1:3; preferably 1:0.7 to 1:2.

In some embodiments, in step c), the reaction solvent is selected from one of DMF, DMAc, NMP, tetrahydrofuran, 1,4-dioxane, acetonitrile, and dichloromethane, or any combination thereof; preferably one of DMF, dichloromethane, and tetrahydrofuran, or any combination thereof.

In some embodiments, in step c), the mass/volume ratio (g/mL) of the compound of formula 6 or the salt thereof to the selected reaction solvent is selected from 1:5 to 1:50.

In some embodiments, in step c), the reaction temperature is selected from -20 to 50°C; preferably -5 to 25°C.

In some embodiments, in step d), the protecting group PG₁ on the amino group is removed from the compound of formula 8 or the salt thereof to obtain the compound of formula 9 or the salt thereof. The step can be carried out in the same way as in step b).

In some embodiments, in step d), when the PG₁ is 9-fluorenylmethoxycarbonyl (Fmoc), the protecting group PG₁ is removed from the compound of formula 8 or the salt thereof in the presence of a base. The base is selected from piperidine, diethylamine, morpholine, diisopropylamine, DBU, triethylamine, and *N,N*-diisopropylethylamine; for example, diethylamine or *N,N-*diisopropylethylamine; preferably piperidine, diethylamine, or morpholine.

In some embodiments, in step d), when the PG₁ is 9-fluorenylmethoxycarbonyl (Fmoc), the reaction solvent is selected from one of DMF, DMAc, dichloromethane, tetrahydrofuran, 1,4-dioxane, acetonitrile, methanol, and ethanol, or any combination thereof.

In some embodiments, in step d), when the PG₁ is 9-fluorenylmethoxycarbonyl (Fmoc), the mass/volume ratio (g/mL) of the compound of formula 8 or the salt thereof to the selected reaction solvent is selected from 1:5 to 1:50.

In some embodiments, in step d), when the PG₁ is 9-fluorenylmethoxycarbonyl (Fmoc), the volume ratio of the selected base to the selected reaction solvent is selected from 1:5 to 1:50.

In some embodiments, in step d), when the PG₁ is 9-fluorenylmethoxycarbonyl (Fmoc), the reaction temperature is selected from -10 to 50°C; preferably 0 to 30°C.

In some embodiments, in step e), when R₃ is hydroxyl, the compound of formula 9 or the salt thereof is reacted with the compound of formula 10 in the presence of a condensing agent to obtain the compound of formula I. The step can be carried out in the same way as in step c).

In some embodiments, in step e), when R₃ is hydroxyl, the condensing agent is selected from DMTMM, HATU, HBTU, HOBt/EDCI, and T₃P; preferably DMTMM or HOBt/EDCI.

In some embodiments, in step e), when R₃ is hydroxyl, the molar ratio of the compound of formula 9 or the salt thereof to the condensing agent is selected from 1:1 to 1:5; preferably 1:1 to 1:2.

In some embodiments, in step e), when R₃ is hydroxyl, the molar ratio of the compound of formula 9 or the salt thereof to the compound of formula 10 is selected from 1:0.8 to 1:2.0.

In some embodiments, in step e), when R₃ is hydroxyl, the reaction solvent is selected from one of DMF, tetrahydrofuran, and dichloromethane, or any combination thereof.

In some embodiments, in step e), when R₃ is hydroxyl, the mass/volume ratio (g/mL) of the compound of formula 9 or the salt thereof to the selected reaction solvent is selected from 1:5 to 1:50.

In some embodiments, in step e), when R₃ is hydroxyl, the reaction temperature is selected from -20 to 50°C; preferably 0 to 30°C.

In some embodiments, in step e), when R₃ is selected from halogen (*e.g.,* chlorine, fluorine, or bromine), it is a hydroxyl group converted through a halogenation reaction with a halogenating reagent.

In some embodiments, in step e), when R₃ is selected from halogen (*e.g.,* chlorine, fluorine, or bromine), the compound of formula 9 or the salt thereof is reacted with the compound of formula 10 in the presence of a base. The base is selected from triethylamine, *N,N*-diisopropylethylamine, DBU, *N*-methylpiperidine, and *N*-methylmorpholine.

In some embodiments, in step e), when R₃ is chlorine, it is a hydroxyl group converted through a chlorination reaction with a chlorinating reagent. The chlorinating reagent is selected from thionyl chloride, oxalyl chloride, and phosphorus oxychloride; preferably thionyl chloride. The chlorination reaction solvent is selected from tetrahydrofuran and dichloromethane; preferably dichloromethane. The chlorination temperature is selected from 0 to 50°C; preferably 0 to 30°C.

In some embodiments, in step e), when R₃ is halogen (*e.g.,* chlorine, fluorine, or bromine), the molar ratio of the compound of formula 9 or the salt thereof to the base described in the reaction is selected from 1:1 to 1:5.

In some embodiments, in step e), when R₃ is halogen (*e.g.,* chlorine, fluorine, or bromine), the molar ratio of the compound of formula 9 or the salt thereof to the compound of formula 10 is selected from 1:0.8 to 1:2.0; preferably 1:1 to 1:1.5.

In some embodiments, in step e), when R₃ is halogen (*e.g.,* chlorine, fluorine, or bromine), the reaction solvent is selected from one of DMF, tetrahydrofuran, and dichloromethane, or any combination thereof.

In some embodiments, in step e), when R₃ is halogen (*e.g.,* chlorine, fluorine, or bromine), the mass/volume ratio (g/mL) of the compound of formula 9 or the salt thereof to the selected reaction solvent is selected from 1:5 to 1:50.

In some embodiments, in step e), when R₃ is halogen (*e.g.,* chlorine, fluorine, or bromine), the reaction temperature is selected from -20 to 50°C; preferably 0 to 30°C.

In some embodiments, in step f-1), the reaction is carried out in the presence of a base. The base is selected from sodium bicarbonate, sodium carbonate, potassium carbonate, cesium carbonate, and potassium bicarbonate; for example, sodium bicarbonate or sodium carbonate; preferably sodium bicarbonate, potassium bicarbonate, or potassium carbonate.

In some embodiments, in step f-1), the reaction solvent is selected from one of tetrahydrofuran, acetone, acetonitrile, 1,4-dioxane, *N,N*-dimethylformamide, *N,N-*dimethylacetamide, dimethyl sulfoxide, and water, or any combination thereof; preferably one of tetrahydrofuran, acetone, acetonitrile, and water, or any combination thereof.

In some embodiments, in step f-1), the mass/volume ratio (g/mL) of the compound of formula 7-A1 or the salt thereof to the selected reaction solvent is selected from 1:5 to 1:20.

In some embodiments, in step f-1), the molar ratio of the compound of formula 7-A1 to the compound of formula 7-A2 or the salt thereof is selected from 1:1 to 1:3.

In some embodiments, in step f-1), the reaction temperature is selected from 0 to 50°C; preferably 0 to 30°C.

In some embodiments, in step f-2), the protecting group PG₃ on the amino group is removed from the compound of formula 7-A3 to obtain the compound of formula 7-A4.

In some embodiments, in step f-2), when the PG₃ is *tert*-butoxycarbonyl (Boc), the protecting group PG₃ is removed from the compound of formula 7-A3 in the presence of an acid. The acid is selected from trifluoroacetic acid, hydrogen chloride, hydrobromic acid, and sulfuric acid; preferably hydrogen chloride or trifluoroacetic acid.

In some embodiments, in step f-2), when the PG₃ is *tert*-butoxycarbonyl (Boc), the reaction solvent is selected from one of tetrahydrofuran, 1,4-dioxane, ethyl acetate, and methyl *tert*-butyl ether, or any combination thereof; preferably tetrahydrofuran or 1,4-dioxane.

In some embodiments, in step f-2), when the PG₃ is *tert*-butoxycarbonyl (Boc), the mass/volume ratio (g/mL) of the compound of formula 7-A3 to the selected reaction solvent is selected from 1:4 to 1:20; preferably 1:5 to 1:15.

In some embodiments, in step f-2), when the PG₃ is *tert-*butoxycarbonyl (Boc), the molar ratio of the compound of formula 7-A3 to the selected acid is selected from 1:5 to 1:50; preferably 1: 10 to 1:30.

In some embodiments, in step f-2), when the PG₃ is *tert*-butoxycarbonyl (Boc), the reaction temperature is selected from 0 to 50°C; preferably 0 to 30°C.

In some embodiments, in step f-3), the compound of formula 7-A4 or the salt thereof undergoes a reductive amination reaction with an aldehyde in the presence of a reducing agent and an acid to obtain the compound of formula 7 or the salt thereof. The reducing agent is selected from sodium borohydride, sodium cyanoborohydride, sodium triacetoxyborohydride, and Pd/C-H₂; preferably sodium cyanoborohydride and/or sodium triacetoxyborohydride. The acid is selected from acetic acid, formic acid, propionic acid, trifluoroacetic acid, and hydrochloric acid; preferably acetic acid or propionic acid.

In some embodiments, in step f-3), the reaction solvent is selected from one of methanol, ethanol, propanol, tetrahydrofuran, 1,4-dioxane, and ethyl acetate, or any combination thereof; preferably methanol or ethanol.

In some embodiments, in step f-3), the mass/volume ratio (g/mL) of the compound of formula 7-A4 or the salt thereof to the selected solvent is selected from 1:5 to 1:20; preferably 1:6 to 1:15.

In some embodiments, in step f-3), the molar ratio of the compound of formula 7-A4 or the salt thereof to the selected reducing agent is selected from 1:2 to 1:10; preferably 1:3 to 1:8.

In some embodiments, in step f-3), the molar ratio of the compound of formula 7-A4 or the salt thereof to the aldehyde is selected from 1:2 to 1:10; preferably 1:4 to 1:8.

In some embodiments, in step f-3), the temperature for adding the reducing agent is selected from -20 to 30°C; preferably 0°C. The reaction temperature is selected from -20 to 50°C; preferably 0 to 30°C.

In some embodiments, in step g-1), the compound of formula 7-B1 or the salt thereof undergoes a reductive amination reaction with an aldehyde in the presence of a reducing agent and an acid to obtain the compound of formula 7-B2 or the salt thereof. The step can be carried out in the same way as in step f-3).

In some embodiments, in step g-1), the reducing agent is selected from sodium borohydride, sodium cyanoborohydride, sodium triacetoxyborohydride, and Pd/C-H₂; preferably sodium cyanoborohydride or sodium triacetoxyborohydride.

In some embodiments, in step g-1), the acid is selected from acetic acid, formic acid, propionic acid, trifluoroacetic acid, and hydrochloric acid; preferably acetic acid or propionic acid.

In some embodiments, in step g-1), the reaction solvent is selected from one of methanol, ethanol, propanol, tetrahydrofuran, 1,4-dioxane, and ethyl acetate, or any combination thereof; preferably methanol or ethanol.

In some embodiments, in step g-1), the mass/volume ratio (g/mL) of the compound of formula 7-B1 or the salt thereof to the selected solvent is selected from 1:5 to 1:20.

In some embodiments, in step g-1), the molar ratio of the compound of formula 7-B1 or the salt thereof to the selected reducing agent is selected from 1:2 to 1:10.

In some embodiments, in step g-1), the molar ratio of the compound of formula 7-B1 or the salt thereof to the aldehyde is selected from 1:2 to 1:10.

In some embodiments, in step g-1), the temperature at which the reducing agent is added is selected from -20 to 30°C. The reaction temperature is selected from -20 to 50°C; preferably 0 to 30°C.

In some embodiments, in step g-2), the protecting group PG₄ on the amino group is removed from the compound of formula 7-B2 or the salt thereof to obtain the compound of formula 7-B3 or the salt thereof. The step can be carried out in the same way as in step b).

In some embodiments, in step g-2), when the PG₄ is 9-fluorenylmethoxycarbonyl (Fmoc), the protecting group PG₄ is removed from the compound of formula 7-B2 or the salt thereof in the presence of a base. The base is selected from piperidine, diethylamine, morpholine, diisopropylamine, DBU, triethylamine, and *N,N*-diisopropylethylamine; preferably piperidine, diethylamine, or morpholine.

In some embodiments, in step g-2), when the PG₄ is 9-fluorenylmethoxycarbonyl (Fmoc), the reaction solvent is selected from one of DMF, DMAc, dichloromethane, tetrahydrofuran, 1,4-dioxane, acetonitrile, methanol, and ethanol, or any combination thereof; preferably one of DMF, tetrahydrofuran, and dichloromethane, or any combination thereof.

In some embodiments, in step g-2), when the PG₄ is 9-fluorenylmethoxycarbonyl (Fmoc), the mass/volume ratio (g/mL) of the compound of formula 7-B2 or the salt thereof to the selected reaction solvent is selected from 1:2 to 1:20.

In some embodiments, in step g-2), when the PG₄ is 9-fluorenylmethoxycarbonyl (Fmoc), the mass/volume ratio (g/mL) of the selected base to the selected reaction solvent is selected from 1:5 to 1:50.

In some embodiments, in step g-2), when the PG₄ is 9-fluorenylmethoxycarbonyl (Fmoc), the reaction temperature is selected from 0 to 50°C; preferably 0 to 30°C.

In some embodiments, in step g-3), the reaction is carried out in the presence of a base. The base is selected from sodium bicarbonate, sodium carbonate, potassium carbonate, cesium carbonate, and potassium bicarbonate; preferably sodium bicarbonate, potassium bicarbonate, or potassium carbonate.

In some embodiments, in step g-3), the reaction solvent is selected from one of tetrahydrofuran, acetone, acetonitrile, 1,4-dioxane, *N,N*-dimethylformamide, *N,N-*dimethylacetamide, dimethyl sulfoxide, and water, or any combination thereof; preferably one of water, tetrahydrofuran, acetone, and acetonitrile, or any combination thereof; more preferably a combination of acetone and water.

In some embodiments, in step g-3), the reaction solvent is selected from a combination of acetone and water, wherein the volume ratio of acetone to water is selected from 1:0.2 to 1:10.

In some embodiments, in step g-3), the molar ratio of the compound of formula 7-B3 or the salt thereof to the compound of 7-A1 is selected from 1:1 to 1:5.

In some embodiments, in step g-3), the reaction temperature is selected from 0 to 50°C.

In some embodiments, in step h), the reaction is carried out in the presence of an acid. The acid is selected from one of *p*-toluenesulfonic acid monohydrate, pyridinium *p-*toluenesulfonate, trifluoroacetic acid, and acetic acid, or any combination thereof; preferably *p*-toluenesulfonic acid monohydrate.

In some embodiments, in step h), the molar ratio of the compound of formula 2 to the selected acid is selected from 1:0.2 to 1:2; preferably 1:0.2 to 1:0.4.

In some embodiments, in step h), the reaction solvent is selected from one of tetrahydrofuran, 1,4-dioxane, toluene, *o*-methylphenol, *N,N*-dimethylformamide, *N,N-*dimethylacetamide, and *N*-methylpyrrolidone, or any combination thereof; preferably one of toluene, *o*-methylphenol, and *N*-methylpyrrolidone, or any combination thereof; more preferably *N*-methylpyrrolidone.

In some embodiments, in step h), the mass/volume ratio (g/mL) of the compound of formula 2 to the selected solvent is selected from 1:1 to 1:60.

In some embodiments, in step h), the molar ratio of the compound of formula 1 or the salt thereof to the compound of formula 2 is selected from 3:1 to 1:1; preferably 2:1 to 1:1.

In some embodiments, in step h), the compound of formula 1 is added to the reaction system in batches; preferably, based on total addition amount, in the presence of an acid, 25 to 35 mol% of the compound of formula 1 or the salt thereof is reacted with the compound of formula 2 at 100 to 120°C, and the remaining compound of formula 1 is added to the reaction system in 2 to 5 batches; more preferably, based on total addition amount, in the presence of an acid, 25 to 35 mol% of the compound of formula 1 or the salt thereof is reacted with the compound of formula 2 at 100 to 120°C for 20 to 40 minutes, and the remaining compound of formula 1 is added to the reaction system in 2 to 5 batches with an interval of 20 to 40 minutes, and after the addition is completed, the reaction is continued for 30 to 90 minutes.

In some embodiments, in step h), the reaction temperature is selected from 70 to 130°C; preferably 80 to 120°C; more preferably 100 to 120°C.

In some embodiments, in step i-1), the compound of formula 1-A1 is reacted with the compound of formula 1-A2 in the presence of a palladium catalyst, a copper catalyst, and a base to obtain the compound of formula 1-A3; the palladium catalyst is selected from Pd(PPh₃)₄, Pd(OAc)₂, Pd(PPh₃)₂Cl₂, and Pd₂(dba)₃, preferably Pd(PPh₃)₂Cl₂; the copper catalyst is selected from CuI, CuBr, and CuCl, preferably CuI; the base is selected from triethylamine, *N,N-*diisopropylethylamine, DBU, potassium carbonate, and cesium carbonate, preferably triethylamine or *N,N*-diisopropylethylamine.

In some embodiments, in step i-1), the molar ratio of the palladium catalyst in the reaction to the compound of formula 1-A1 is selected from 0.01:1 to 0.2:1; preferably 0.01:1 to 0.10:1; more preferably 0.01:1 to 0.03:1.

In some embodiments, in step i-1), the molar ratio of the copper catalyst in the reaction to the compound of formula 1-A1 is selected from 0.01:1 to 0.2:1; preferably 0.02:1 to 0.10:1; more preferably 0.02:1 to 0.05:1.

In some embodiments, in step i-1), the molar ratio of the compound of formula 1-A1 to the compound of formula 1-A2 is selected from 1:1 to 1:5; preferably 1:1 to 1:3; more preferably 1:1 to 1:2.

In some embodiments, in step i-1), the molar ratio of the base in the reaction to the compound of formula 1-A1 is selected from 10:1 to 1:1; preferably 5:1 to 2:1.

In some embodiments, in step i-1), the reaction solvent is selected from one of tetrahydrofuran, 1,4-dioxane, toluene, *N*-methylpyrrolidone, *N,N*-dimethylformamide, and *N,N*-dimethylacetamide, or any combination thereof; for example, the reaction solvent is selected from one of tetrahydrofuran, 1,4-dioxane, toluene, *N,N*-dimethylformamide, and *N,N-*dimethylacetamide, or any combination thereof; for another example, tetrahydrofuran, *N-*methylpyrrolidone, or *N,N*-dimethylformamide; preferably tetrahydrofuran or 1,4-dioxane.

In some embodiments, in step i-1), the mass/volume ratio (g/mL) of the compound of formula 1-A1 to the selected solvent is selected from 1:5 to 1:20; preferably 1:10 to 1:20.

In some embodiments, in step i-1), the reaction temperature is selected from 40 to 80°C; preferably 40 to 60°C.

In some embodiments, in step i-2), the compound of formula 1-A3 is reacted in the presence of Sn and Na₂S to obtain the compound of formula 1 or the salt thereof; Na₂S is added to the system in the form of Na₂S·2H₂O and/or Na₂S·9H₂O.

In some embodiments, in step i-2), the molar ratio of the Sn to the compound of formula 1-A3 is selected from 5:1 to 1:1; preferably 3:1 to 1:1.

In some embodiments, in step i-2), the molar ratio of the Na₂S to the compound of formula 1-A3 is selected from 0.1:1 to 2:1; preferably 0.2:1 to 1:1.

In some embodiments, in step i-2), the reaction solvent is selected from one of tetrahydrofuran, acetone, acetonitrile, 1,4-dioxane, methanol, ethanol, propanol, and water, or any combination thereof; preferably one of tetrahydrofuran, ethanol, and methanol, or any combination thereof; more preferably a combination of ethanol and water, wherein the volume ratio of ethanol to water is selected from 20:1 to 1:1, preferably 10:1 to 5:1.

In some embodiments, in step i-2), the reaction temperature is selected from 40 to 100°C; for example, 50 to 80°C; preferably 60 to 90°C.

In some embodiments, in step j-1), the compound of formula 1-B1 undergoes a Grignard reaction with the compound of formula 1-B2 to obtain the compound of formula 1-B3.

In some embodiments, in step j-1), the molar ratio of the compound of formula 1-B1 to the compound of formula 1-B2 is selected from 1:0.8 to 1:2; preferably 1:1 to 1:1.5.

In some embodiments, in step j-1), the reaction solvent is selected from one of tetrahydrofuran, 2-methyltetrahydrofuran, diisopropyl ether, and diethyl ether, or any combination thereof; preferably tetrahydrofuran or diisopropyl ether.

In some embodiments, in step j-1), the reaction temperature is selected from -20 to 60°C; preferably -20 to 50°C; more preferably 0 to 30°C.

In some embodiments, in step j-2), the compound of formula 1-B3 undergoes a nitration reaction to obtain the compound of formula 1-B4.

In some embodiments, in step j-2), the nitration reaction system is selected from concentrated nitric acid, concentrated nitric acid/acetic acid, and concentrated sulfuric acid/potassium nitrate.

In some embodiments, in step j-2), the mass/volume ratio (g/mL) of the compound of formula 1-B3 to the nitration system is selected from 1:4 to 1:20; preferably 1:6 to 1:15.

In some embodiments, in step j-2), the reaction temperature is selected from -20 to 30°C; preferably -20 to 0°C.

In some embodiments, in step j-3), the nitro group of the compound of formula 1-B4 is reduced to an amino group to obtain the compound of formula 1-B5 or the salt thereof; the reaction can be carried out under conventional reaction conditions well known to those skilled in the art.

In some embodiments, in step j-3), the reducing agent for the reaction is selected from hydrogen, TiCl₃, SnCl₂, sodium dithionite, zinc powder, and iron powder; preferably hydrogen, SnCl₂, or sodium dithionite.

In some embodiments, in step j-3), the reaction is carried out under hydrogen atmosphere with the addition of a transition metal catalyst. The transition metal catalyst is selected from platinum dioxide, palladium on carbon, rhodium on carbon, and Raney nickel.

In some embodiments, in step j-3), when the reaction is carried out under a hydrogen-platinum dioxide system, the mass ratio of the compound of formula 1-B4 to platinum dioxide is selected from 1:0.01 to 1:0.4; preferably 1:0.01 to 1:0.1.

In some embodiments, in step j-3), when the reaction is carried out under the hydrogen-platinum dioxide system, hydrogen pressure is selected from 1 to 10 atmospheres, preferably 1 to 5 atmospheres.

In some embodiments, in step j-3), when the reaction is carried out under the hydrogen-platinum dioxide system, the reaction temperature is selected from -10 to 100°C; preferably -10 to 80°C.

In some embodiments, in step j-3), when the reaction is carried out under the hydrogen-platinum dioxide system, the reaction solvent is selected from one of methanol, ethanol, ethyl acetate, tetrahydrofuran, and 1,4-dioxane, or any combination thereof; preferably ethyl acetate or tetrahydrofuran.

In some embodiments, in step j-4), the reaction is carried out in the presence of a base. The base is selected from potassium carbonate, potassium hydroxide, sodium hydroxide, cesium carbonate, and sodium carbonate; for example, the base is selected from potassium carbonate, sodium hydroxide, cesium carbonate, and sodium carbonate; for another example, potassium carbonate or potassium hydroxide; preferably potassium carbonate or sodium carbonate.

In some embodiments, in step j-4), the molar ratio of the compound of formula 1-B5 to the base is selected from 1:1 to 1:5; preferably 1:1 to 1:3.

In some embodiments, in step j-4), the reaction solvent is selected from one of water, tetrahydrofuran, 1,4-dioxane, acetonitrile, hexamethylphosphoramide, *N,N-*dimethylformamide, *N,N*-dimethylacetamide, *N*-methylpyrrolidone, and dimethyl sulfoxide, or any combination thereof; preferably one of water, acetonitrile, tetrahydrofuran, dimethyl sulfoxide, and hexamethylphosphoramide, or any combination thereof.

In some embodiments, in step j-4), the reaction solvent is selected from a combination of acetonitrile and water, wherein the volume ratio of acetonitrile to water is selected from 1:5 to 10:1; preferably 1:2 to 5:1.

In some embodiments, in step j-4), the mass/volume ratio (g/mL) of the compound of formula 1-B5 or the salt thereof to the reaction solvent is selected from 1:5 to 1:20; preferably 1:5 to 1:10.

In some embodiments, in step j-4), the reaction temperature is selected from 20 to 100°C; preferably 40 to 80°C.

### Intermediate

In another aspect, the present application provides the compound of formula 1-B4, the compound of formula 1-B5 or the salt thereof, the compound of formula 1 or the salt thereof, the compound of formula 7 or the salt thereof, the compound of formula 8 or the salt thereof, the compound of formula 9 or the salt thereof as follows, 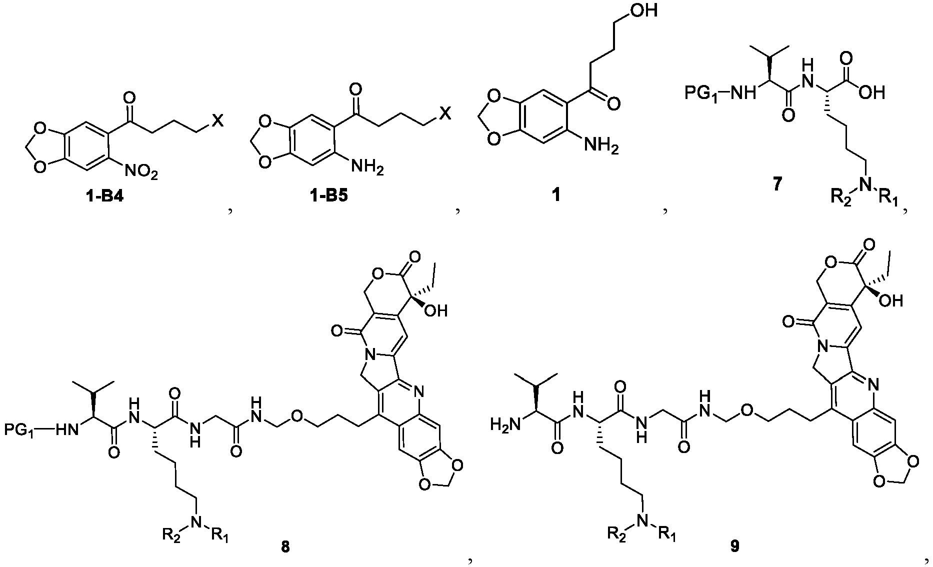 wherein
R₁ and R₂ are each independently selected from C₁₋₄ alkyl;
X is selected from halogen;
PG₁ is selected from an amino protecting group;
the compound of formula 7 is not

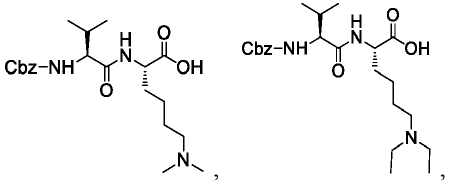

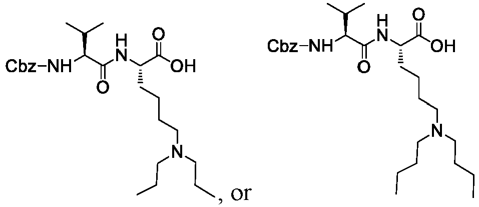

In another aspect, the present application provides a use of the compound of formula 1-B4 or the salt thereof, the compound of formula 1-B5 in the preparation of the compound of formula 1 or the salt thereof, the compound of formula 3 or the salt thereof, or the compound of formula I or the salt thereof, 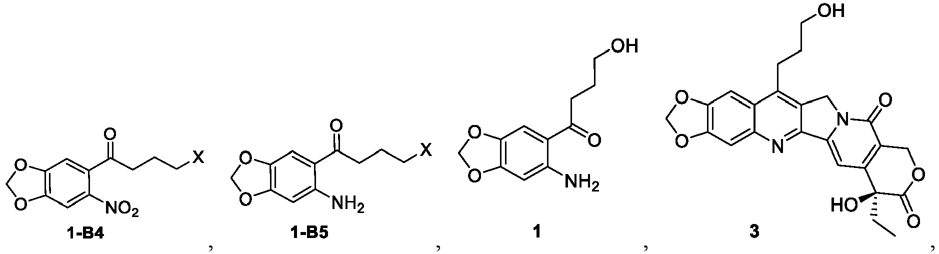 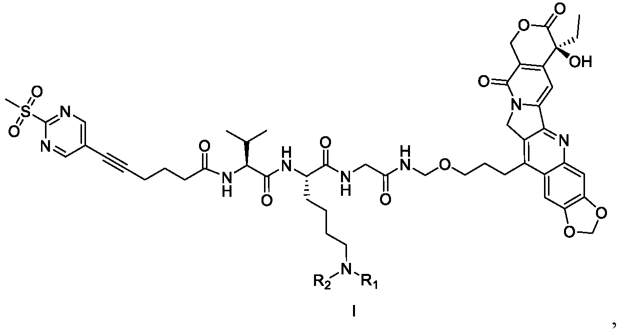 wherein
R₁ and R₂ are each independently selected from C₁₋₄ alkyl;
X is selected from halogen.

In another aspect, the present application provides a use of the compound of formula 1 or the salt thereof in the preparation of the compound of formula 3 or the salt thereof or the compound of formula I or the salt thereof, 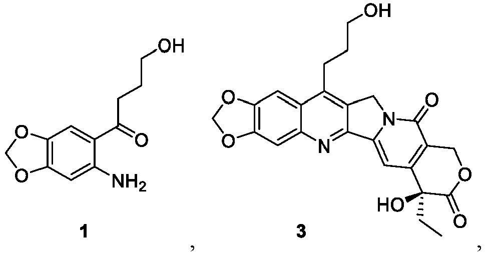 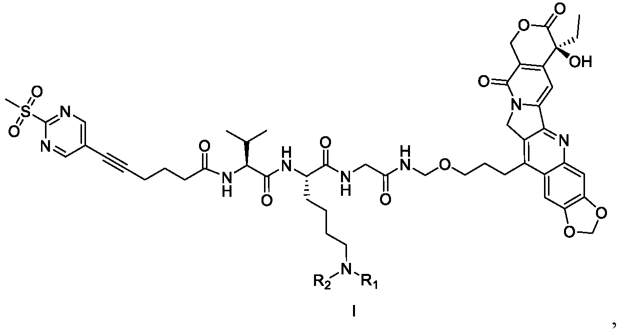 wherein
R₁ and R₂ are each independently selected from C₁₋₄ alkyl.

In another aspect, the present application provides a use of the compound of formula **7** or the salt thereof, the compound of formula **8** or the salt thereof, or the compound of formula **9** or the salt thereof in the preparation of the compound of formula I, 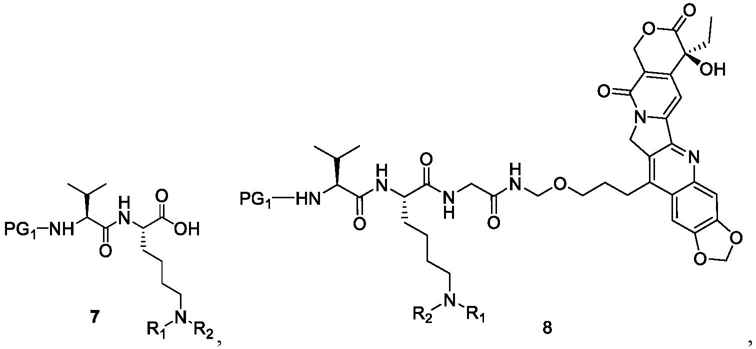 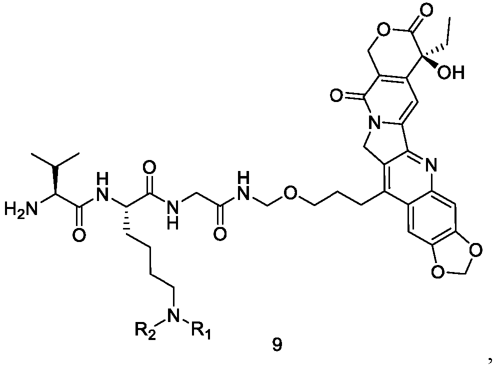 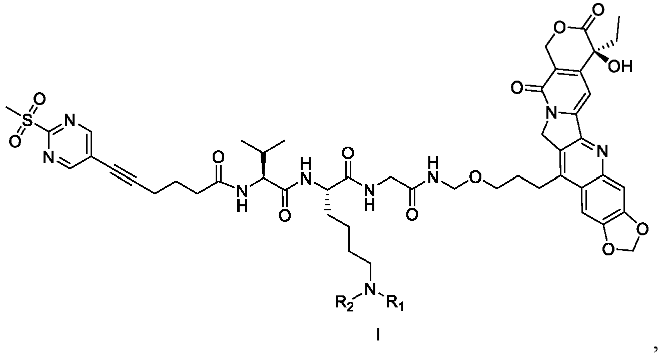 wherein
R₁ and R₂ are each independently selected from C₁₋₄ alkyl;
PG₁ is selected from an amino protecting group.

In some embodiments, in the intermediate or the use thereof, the R₁ and R₂ are each independently selected from methyl, ethyl, and propyl, preferably R₁ and R₂ are the same.

In some embodiments, in the intermediate or the use thereof, both R₁ and R₂ are propyl.

In some embodiments, in the intermediate or the use thereof, the X is selected from fluorine, chlorine, bromine, and iodine.

In some embodiments, in the intermediate or the use thereof, the X is chlorine.

In some embodiments, the PG₁ is selected from an alkoxycarbonyl protecting group; preferably benzyloxycarbonyl (Cbz), 9-fluorenylmethoxycarbonyl (Fmoc), allyloxycarbonyl (Alloc), (trimethylsilyl)ethoxycarbonyl (Teoc), methoxycarbonyl, or ethoxycarbonyl; more preferably 9-fluorenylmethoxycarbonyl (Fmoc).

In some embodiments of the present application, the compound of formula 1-B4 is selected from:

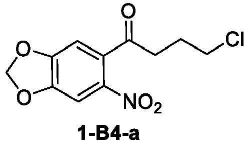

In some embodiments of the present application, the compound of formula 1-B5 or the salt thereof is selected from:

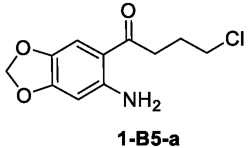

In some embodiments of the present application, the compound of formula **7** or the salt thereof is selected from:

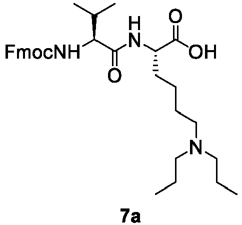

In some embodiments of the present application, the compound of formula **8** or the salt thereof is selected from:

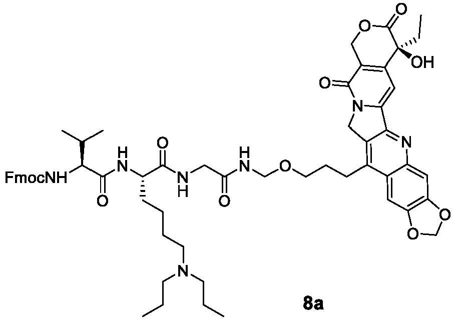

In some embodiments of the present application, the compound of formula **9** or the salt thereof is selected from:

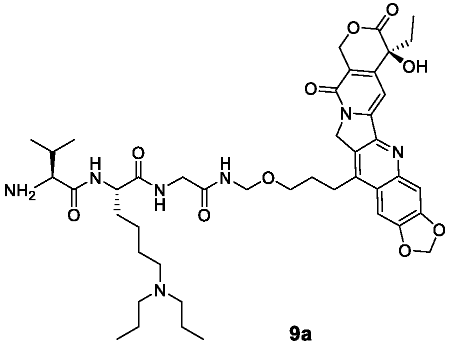

### Definition and terminology

Unless otherwise specified, the terms and phrases used herein have the following meanings. A specific term or phrase should not be considered indefinite or unclear in the absence of a particular definition, but should be interpreted in accordance with the meaning commonly understood by those skilled in the art. When a trade name appears herein, it is intended to refer to its corresponding commodity or active ingredient thereof.

In the present application, the singular form of a word includes the plural form, unless otherwise specified or implied by the context. Thus, references to singular terms and "the" are generally inclusive of the plurals of the respective terms. The terms "include" and "comprise" should be construed as inclusive and not exclusive.

In the present application, the term "salt thereof" as used herein refers to the salt form of a compound (e.g., a compound of formula 1), unless otherwise specified or implied by the context. The salt form of a compound has one or more than one internal salt form and/or involves the inclusion of another molecule, such as an acetate ion, a succinate ion, or other counterion. The counterion in the salt form of a compound is typically an organic or inorganic moiety that stabilizes the charge on the parent compound. The salt form of a compound has one or more charged atom in its structure. In the case where multiple charged atoms are part of the salt form, multiple counterions and/or multiple charged counterions are present. Thus, the salt form of a compound typically has one or more charged atom corresponding to the non-salt form of the compound and/or one or more counterion. In some aspects, the non-salt form of a compound contains at least one amino group or other basic moiety, and accordingly in the presence of an acid, an acid addition salt having a basic moiety is obtained. In other aspects, the non-salt form of a compound contains at least one carboxylic acid group or other acidic moiety, and accordingly in the presence of a base, a carboxylate or other anionic moiety is obtained.

In the present application, exemplary counteranions and countercations in the salt form of a compound include, but are not limited to, sulfate, trifluoroacetate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, *p*-toluenesulfonate, and pamoate (*i.e.,* 1,1'-methylene-bis(2-hydroxy-3-naphthoate)).

In the present application, the term "C₁₋₄ alkyl" refers to a linear or branched alkyl group containing 1 to 4 carbon atoms. Specific examples include, but are not limited to, methyl, ethyl, *n*-propyl, isopropyl, 1-*n*-butyl, 2-*n*-butyl, isobutyl, *tert*-butyl, *etc.*

In the present application, the term "room temperature" refers to 25 ± 5°C.

In the present application, the protecting group and the method for attaching or removing the same can be achieved by using a conventional method in the art, and the method can be one-step reaction or multi-step reaction, such as, but not limited to, referring to "Greene's Protective Groups in Organic Synthesis - 4th Edition" published by Wiley Press, or "Protecting Groups" published by Chemical Industry Press.

In the present application, the term "amino protecting group" refers to a group that protects the amino group in a compound and may be selected from amino protecting groups known in the art. The medium for the protection reaction described in the present application is preferably selected from an aprotic solvent, preferably dichloromethane. The upper protecting group reaction for the protection of the amino group can be carried out using conventional reaction conditions well known to those skilled in the art. The reaction for removing the protecting group from the amino group can be carried out using conventional reaction conditions well known to those skilled in the art. Preferably, the amino protecting group used in the present application is selected from an alkoxycarbonyl protecting group, which may be unsubstituted or substituted, such as benzyloxycarbonyl, *tert*-butoxycarbonyl, fluorenylmethoxycarbonyl, allyloxycarbonyl, (trimethylsilyl)ethoxycarbonyl, methoxycarbonyl, or ethoxycarbonyl.

In the present application, when the term "approximately" is used in conjunction with a numerical value or numerical range, it modifies the numerical value or numerical range by extending the boundaries of the numerical value upwards and/or downwards. For example, the term "approximately" is intended to modify a numerical value by ≤ 20%, preferably ≤ 10%, above and below the stated value.

In the present application, if both the name and the structural formula of a compound are given with respect to the compound, in the event of inconsistency between the two, the structure of the compound prevails, unless the context indicates that the structure of the compound is incorrect but the name is correct.

In the present application, compounds may exist in specific geometric or stereoisomeric forms. The present application contemplates all such compounds, including *cis* and *trans* isomers, (-)- and (+)-enantiomers, (*R*)- and (*S*)-enantiomers, diastereomers, (*D*)-isomers, (*L*)-isomers, racemic and other mixtures thereof, such as enantiomers or diastereomeric enriched mixtures, all of which are within the scope of the present application. Additional asymmetric carbon atoms may be present in substituents such as alkyl. All these isomers and their mixtures are comprised within the scope of the present application.

The reagents and raw materials used in the present application are all commercially available.

The positive and progressive effect of the present application lies in the fact that the raw materials for the preparation method of the present application are readily available and low in price. The purification of intermediates and products are easy to operate. The reaction is carried out at normal pressure in mild conditions, and the reaction temperature is easy to control. The purity of the final product is high. Also, the preparation method of the present application can be scaled up and is suitable for large-scale synthesis. Moreover, the drug linker conjugate prepared in the present application can be conjugated with an antibody to obtain an antibody-drug conjugate (ADC), which has good anti-tumor activity.

### DETAILED DESCRIPTION OF THE PREFERRED EMBODIMENT

### Sequence information

Information on the sequences involved in the present application is described in the table below.

| SEQ ID NO | Description | Sequence |
|---|---|---|
| 1 | Heavy chain (HC) of 2E3-02 | EVQLLESGGGLVQPGGSLRLSCAASGFTFSSYAMNWVRQAPGK GLEWVSGISGSGGSTYYADSVQGRFTISRDNSKNTLYLQMNSLR AEDTAVYYCAKARSGYDYFFDYWGQGTLVTVSSASTKGPSVFPL APSSKSTSGGTAALGCLVKDYFPEPVTVSWNSGALTSGVHTFPAV LQSSGLYSLSSVVTVPSSSLGTQTYICNVNHKPSNTKVDKKVEPK SCDKTHTCPPCPAPELLGGPSVFLFPPKPKDTLMISRTPEVTCVVV DVSHEDPEVKFNWYVDGVEVHNAKTKPREEQYNSTYRVVSVLT VLHQDWLNGKEYKCKVSNKALPAPIEKTISKAKGQPREPQVYTL PPSRDELTKNQVSLTCLVKGFYPSDIAVEWESNGQPENNYKTTPP VLDSDGSFFLYSKLTVDKSRWQQGNVFSCSVMHEALHNHYTQK SLSLSPGK |
| 2 | Light chain (LC) of 2E3-02 | EIVLTQSPGTLSLSPGERATLSCRASQRISSSFLAWYQQKPGQAPS LLIYGASSRATGIPDRFSGSGSGTDFTLTISSLEPEDFAVFYCQQYS NSPLTFGGGTKVEIKRTVAAPSVFIFPPSDEQLKSGTASVVCLLNN FYPREAKVQWKVDNALQSGNSQESVTEQDSKDSTYSLSSTLTLS KADYEKHKVYACEVTHQGLSSPVTKSFNRGEC |

All features disclosed in this specification, or steps in all methods or processes disclosed, may be combined in any manner, except for mutually exclusive features and/or steps.

Any feature disclosed in this specification, unless otherwise specifically stated, may be replaced by alternative features which are equivalent or serve a similar purpose. That is, unless otherwise specifically stated, each feature is only one example of a series of equivalent or similar features.

The implementation conditions used in the examples can be further adjusted according to specific requirements, and the unspecified implementation conditions are usually those used in routine experiments.

Among them, the chemical reagents used in the following examples are all commercially available chemical reagents.

In the conventional synthesis methods and examples as well as intermediate synthesis examples, the meaning of each abbreviation or English word is shown in the table below:

| | | | |
|---|---|---|---|
| Cbz | Benzyloxycarbonyl | NaBH₄ | Sodium borohydride |
| Fmoc | 9-Fluorenylmethoxycarbonyl | NaCNBH₃ | Sodium cyanoborohydride |
| Alloc | Allyloxycarbonyl | NaBH(OAc)₃ | Sodium triacetoxyborohydride |
| Teoc | (Trimethylsilyl)ethoxycarbonyl | HCl | Hydrogen chloride |
| Boc | *tert*-Butoxycarbonyl | PTSA | *p*-Toluenesulfonic acid monohydrate |
| DMTMM | 4-(4,6-Dimethoxytriazin-2-yl)-4-methylmorpholine hydrochloride | TFA | Trifluoroacetic acid |
| HATU | 2-(7-Azabenzotriazol-1-yl)-*N,N,N',N'-*tetramethyluronium hexafluorophosphate | AcOH | Acetic acid |
| HBTU | O-(benzotriazol-1-yl)-*N,N,N',N'-*tetramethyluronium hexafluorophosphate | Pd(PPh₃)₄ | Tetrakis(triphenylphosphine)palladium |
| HOBt | 1 -Hydroxybenzotriazole | Pd(OAc)₂ | Palladium acetate |
| EDCI | 1-Ethyl-(3-dimethylaminopropyl)carbodiimide hydrochloride | Pd(PPh₃)Cl₂ | Bis(triphenylphosphine)palladium chloride |
| T₃P | Propylphosphonic anhydride | Pd₂(dba)₃ | Tris(dibenzylideneacetone)dipalladium |
| NaHCO₃ | Sodium bicarbonate | CuI | Cuprous iodide |
| Na₂CO₃ | Sodium carbonate | CuBr | Cuprous bromide |
| K₂CO₃ | Potassium carbonate | CuCl | Cuprous chloride |
| Cs₂CO₃ | Cesium carbonate | Et₃N | Triethylamine |
| KHCO₃ | Potassium bicarbonate | DIPEA | *N,N*-Diisopropylethylamine |
| THF | Tetrahydrofuran | DBU | 1,8-Diazabicyclo[5.4.0]undec-7-ene |
| Acetone | Acetone | PbO₂ | Platinum dioxide |
| CH₃CN | Acetonitrile | Pd/C | Palladium on carbon |
| 1,4-Dioxane | 1,4-Dioxane | Rh/C | Rhodium on carbon |
| DMF | *N,N-*Dimethylformamide | Raney nickel | Raney nickel |
| DMAc | *N,N*-Dimethylacetamide | TiCl₃ | Titanium trichloride |
| DMSO | Dimethyl sulfoxide | SnCl₂ | Stannous chloride |
| HMPA | Hexamethylphosphoramide | Na₂S₂O₄ | Sodium dithionite |
| NMP | *N*-methylpyrrolidone | Fe | Zinc powder |
| BCl₃ | Boron trichloride | Zn | Iron powder |
| DCE | 1,2-Dichloroethane | PE | Petroleum ether |
| DCM | Dichloromethane | EA | Ethyl acetate |
| MeOH | Methanol | EtOH | Ethanol |
| MTBE | Methyl *tert*-butyl ether | h | Hour |

In an exemplary embodiment of the present application, compound 1 is synthesized using the following route:

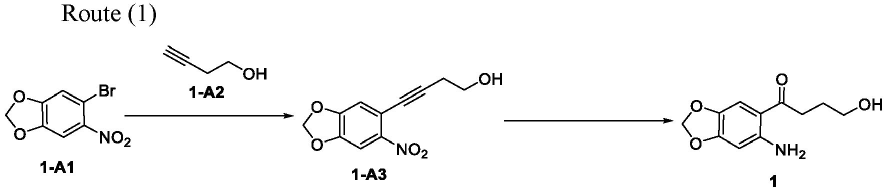

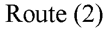

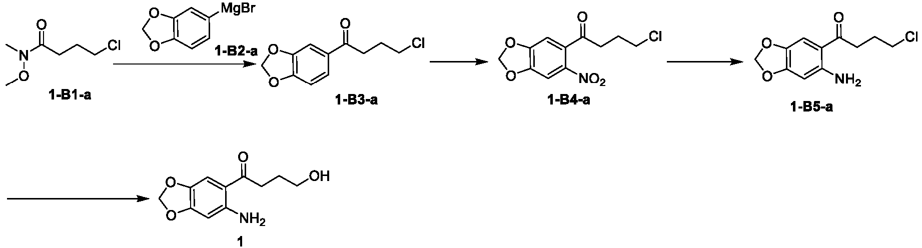

In an exemplary embodiment of the present application, compound 3 is synthesized using the following route:

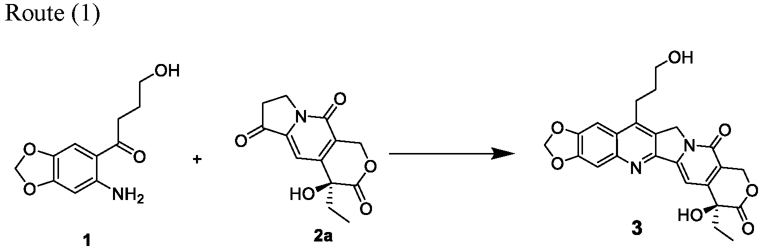

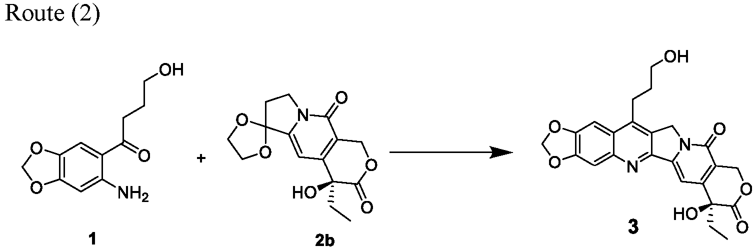

In an exemplary embodiment of the present application, compound 7a is synthesized using the following route:

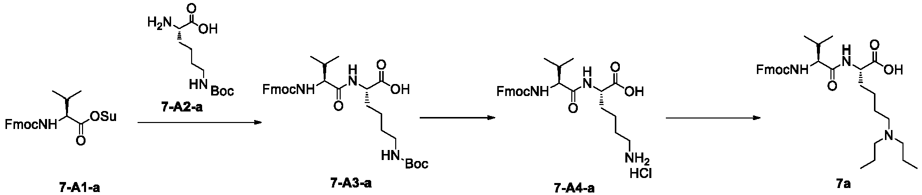

In an exemplary embodiment of the present application, drug linker conjugate **Ia** is synthesized using the following route:

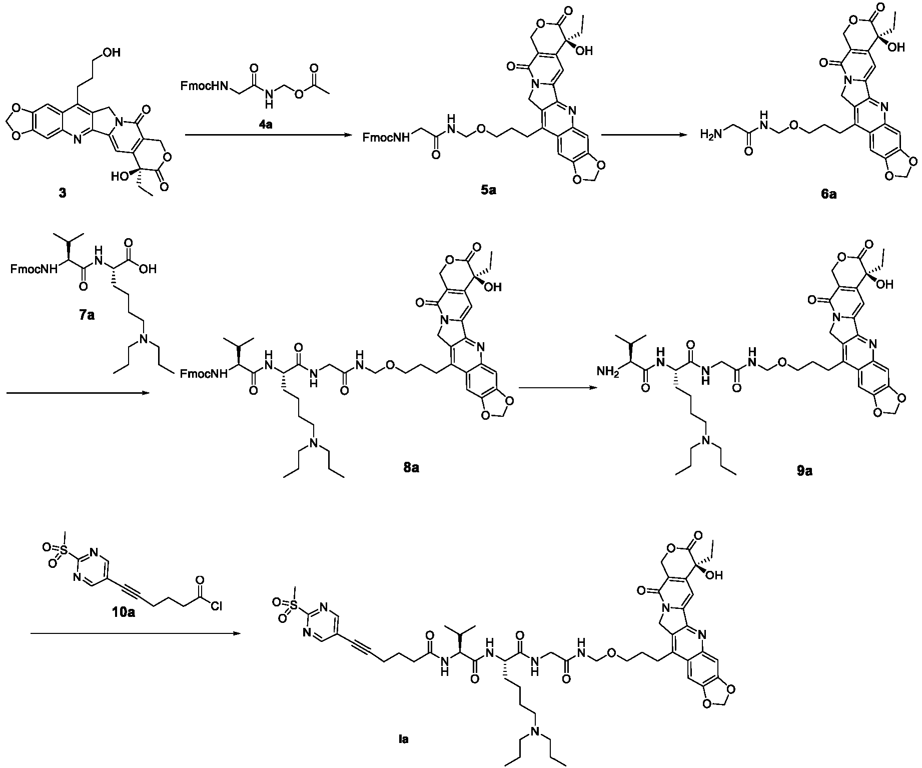

### Example 1: Preparation of 4-(6-nitrobenzo[d][1,3]dioxol-5-yl)but-3-yn-1-ol (compound 1-A3)

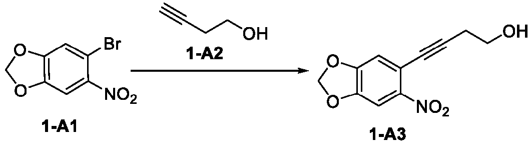

Method 1: Compounds **1-A1** (100 g, 408 mmol) and **1-A2** (57.2 g, 816 mmol) were dissolved in THF (1.5 L), then CuI (3.88 g, 20.4 mmol), Pd(PPh₃)₂Cl₂ (8.60 g, 12.24 mmol), and triethylamine (102 g, 1.02 mol, 141 mL) were added thereto, and the reaction mixture was reacted at 50°C overnight under nitrogen atmosphere. The reaction was monitored by LCMS. The reaction mixture was filtered through diatomite while hot, and the filtrate was concentrated to obtain a crude product, which was then slurried with ethyl acetate (300 mL) to obtain the target compound **1-A3** (65 g, yield: 67%, purity: 98.9%).

Method 2: Compounds **1-A1** (100 g, 408 mmol) and **1-A2** (11.4 g, 163 mmol) were dissolved in NMP (300 mL), then triethylamine (102 g, 1.02 mol, 141 mL) was added thereto, and the reaction mixture was placed under nitrogen atmosphere. CuI (3.88 g, 20.4 mmol) and Pd(PPh₃)₂Cl₂ (2.9 g, 4.1 mmol) were then sequentially added thereto, and the reaction mixture was reacted at 50°C for 0.5 hours. **1-A2** (22.8 g, 326 mmol) was added dropwise thereto, and after the dropwise addition was completed, the reaction mixture was reacted at 50°C for another 4 hours. The reaction mixture was cooled to room temperature, then poured into water (4.5 L) containing ammonia water (3.26 mol) to precipitate a solid, and stirred for 0.5 hours. After filtration under reduced pressure, the filter cake was added with water (4.5 L) and stirred for 0.5 hours. After filtration under reduced pressure, the filter cake was added with water (4.5 L) and stirred for 0.5 hours. The filter cake was dried to obtain the target compound **1-A3** (85.0 g, yield: 88%, purity: 96.4%).

Method 3: Compounds **1-A1** (100 g, 408 mmol) and **1-A2** (34.1 g, 490 mmol) were dissolved in DMF (0.5 L), and CuI (1.55 g, 8.16 mmol) and Pd(PPh₃)₂Cl₂ (2.87 g, 4.08 mmol) were added thereto. Triethylamine (204 g, 2.02 mol) was added dropwise thereto, and after the dropwise addition was completed, the reaction mixture was reacted at 50°C for 3 hours. The reaction mixture was cooled to no more than 30°C, added to 1% ammonia water (1L), and stirred for 30 minutes. The resulting mixture was filtered under reduced pressure, and the filter cake was slurried with water (500 mL) for 20 minutes, and then filtered under reduced pressure. The filter cake was dried to obtain the target compound **1-A3** (86 g, yield: 90%, purity: 96.6%).

Compound **1-A3:** LCMS (ESI) [M+H]⁺ = 236.0.

Compound **1-A3:** ¹H NMR (400 MHz, DMSO-d₆) δ 7.66 (s, 1H), 7.18 (s, 1H), 6.25 (s, 2H), 4.91 (t, *J =* 5.6 Hz, 1H), 3.59 (dd, *J =* 12.6, 6.7 Hz, 2H), 2.59 (t, *J =* 6.9 Hz, 2H).

### Example 2: Preparation of 1-(6-aminobenzo[d][1,3]dioxol-5-yl)-4-hydroxybutan-1-one (compound 1)

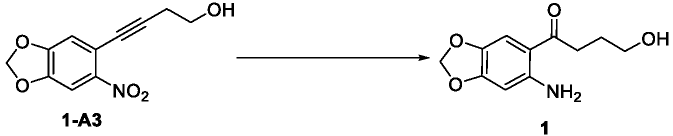

Method 1: Compound **1-A3** (8.42 g, 35.79 mmol) was dissolved in a solution of EtOH and H₂O with a ratio of 9:1 (v:v, 152 mL), and Sn (8.46 g, 71.58 mmol) and Na₂S·2H₂O (0.84 g, 10.74 mmol) were added thereto. Concentrated hydrochloric acid (30 mL, 358 mmol) was then added thereto, and the reaction mixture was stirred at 78°C for 1 hour. The reaction was monitored by LCMS. The reaction mixture was filtered under reduced pressure through diatomite while hot, and the filtrate was concentrated to obtain a crude product. The reaction mixture was slurried with ethyl acetate (20 mL), then filtered under reduced pressure, and the filter cake was washed twice with ethyl acetate (5 mL) to obtain a crude product of compound **1.** The crude product of compound **1** (5.0 g) was added with water (50 mL), then added dropwise with saturated sodium bicarbonate solution to adjust the pH to approximately 8.0, and stirred at room temperature for 1 hour. The reaction mixture was filtered under reduced pressure to obtain a filter cake and a filtrate. The filtrate was then extracted with ethyl acetate (50 mL * 2) and concentrated to obtain a solid. The concentrated solid and the filter cake were dried to obtain compound **1** (3.55 g, yield: 51%, purity: 99.7%).

Method 2: Compound **1-A3** (80 g, 340 mmol) was dissolved in a solution of EtOH and H₂O with a ratio of 9:1 (v:v, 1600 mL), and Sn (80.4 g, 680 mmol) and Na₂S·9H₂O (24.5 g, 102 mmol) were added thereto. Concentrated hydrochloric acid (142 mL, 1.7 mol) was then added thereto, and the reaction mixture was stirred at 55°C for 1 hour. The reaction mixture was filtered under reduced pressure through diatomite while hot, and rinsed with a small amount of ethanol. The filtrates were combined, concentrated to dryness, added with ethyl acetate (800 mL) to dissolve, and slowly diluted into sodium carbonate aqueous solution to finally adjust the pH to 8. After filtration, the filtrate was collected, and the phases were separated. The organic phase was concentrated to precipitate a large amount of brown solid, cooled to room temperature, filtered under reduced pressure, and dried to obtain compound **1** (50.1 g, yield: 66.0%, purity: 93.2%).

Compound **1:** LCMS (ESI) [M-18+H]⁺ = 206.2.

Compound **1:** ¹H NMR (400 MHz, CD₃OD) δ 7.18 (s, 1H), 6.24 (s, 1H), 5.87 (s, 2H), 3.62 (t, *J =* 6.7 Hz, 2H), 2.95 - 2.84 (m, 2H), 1.95 - 1.82 (m, 2H).

### Example 3: Preparation of 1-(benzo[d][1,3]dioxol-5-yl)-4-chlorobutan-1-one (compound 1-B3-a)

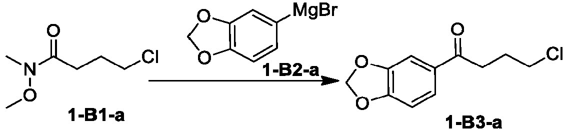

Method 1: Magnesium chips (12.60 g, 518 mmol), iodine particles (0.50 g), and magnets were added to a reaction flask. A reflux condenser was installed, then THF (200 mL) was added thereto under nitrogen atmosphere, and the stirring was started. Firstly, 4-bromo-1,2-methylenedioxybenzene (10.00 g, 50 mmol, 0.12 eq) was added thereto, then heated with hot air from a hair dryer to initiate a reaction, and the color of the reaction system changed from light yellow to colorless. THF (300 mL) was again added thereto, and 4-bromo-1,2-methylenedioxybenzene (90.00 g, 450 mmol, 1.08 eq) was added thereto in batches to keep the reaction system slightly boiling. Most of the magnesium chips were consumed. The heat release in the reaction was stopped and the system was naturally returned to room temperature. The newly prepared Grignard reagent **1-B2-a** was left for further use.

Compound **1-B1-a** (68.66 g, 415 mmol), THF (500 mL), and magnets were added to the reaction flask. The reaction system was cooled to 0 to 5°C, and the stirring was started. A solution of the above prepared Grignard reagent **1-B2-a** in THF was slowly added dropwise thereto through a constant pressure addition funnel to maintain the system temperature at 0 to 10°C. After the addition was completed, the system was returned to room temperature and reacted overnight.

After the reaction was completed, the reaction mixture was added with saturated ammonium chloride aqueous solution (40 mL) to quench the reaction, and diluted with EtOAc (1000 mL) and saturated brine (1500 mL). The reaction mixture was added with 2 M HCl (250 mL) to adjust the pH to 6 to 7, shaken to separate the phases, dried over anhydrous sodium sulfate, filtered, and subjected to rotary evaporation to remove the solvent to obtain a crude product. The crude product was dissolved in methyl *tert*-butyl ether (80 mL), and slowly added with *n*-heptane (200 mL) under stirring to precipitate a solid. The system was cooled to -30°C, stirred for 15 minutes, filtered under reduced pressure at low temperature, and the solid was transferred and dried to obtain compound **1-B3-a** (87.21 g, yield: 92%, purity: 96.8%).

Method 2: Magnesium chips (14.71 g, 613 mmol), iodine particles (0.58 g), and magnets were added to a reaction flask. A reflux condenser was installed, then diisopropyl ether (200 mL) was added thereto under nitrogen atmosphere, and the stirring was started. Firstly, 4-bromo-1,2-methylenedioxybenzene (11.66 g, 58 mmol, 0.14 eq) was added thereto, then heated with hot air from a hair dryer to initiate a reaction, and the color of the reaction system changed from light yellow to colorless. Diisopropyl ether (300 mL) was again added thereto, and 4-bromo-1,2-methylenedioxybenzene (105.1 g, 523 mmol, 1.26 eq) was added thereto in batches to keep the reaction system slightly boiling. Most of the magnesium chips were consumed. The heat release in the reaction was stopped and the system was naturally returned to room temperature. The newly prepared Grignard reagent **1-B2-a** was left for further use.

Compound **1-B1-a** (68.66 g, 415 mmol), diisopropyl ether (500 mL), and magnets were added to the reaction flask. The reaction system was cooled to 0 to 5°C, and the stirring was started. A solution of the above prepared Grignard reagent **1-B2-a** in diisopropyl ether was slowly added dropwise thereto through a constant pressure addition funnel to maintain the system temperature at 0 to 10°C. After the addition was completed, the system was returned to room temperature and reacted overnight.

After the reaction was completed, the reaction mixture was added with saturated ammonium chloride aqueous solution (40 mL) to quench the reaction, and diluted with EtOAc (1000 mL) and saturated brine (1500 mL). The reaction mixture was added with 2 M HCl (250 mL) to adjust the pH to 6 to 7, shaken to separate the phases, dried over anhydrous sodium sulfate, filtered, and subjected to rotary evaporation to remove the solvent to obtain a crude product. The crude product was dissolved in methyl *tert*-butyl ether (80 mL), and slowly added with *n*-heptane (200 mL) under stirring to precipitate a solid. The system was cooled to -30°C, stirred for 15 minutes, filtered under reduced pressure at low temperature, and the solid was transferred and dried to obtain compound **1-B3-a** (86.52 g, yield: 91.3%, purity: 95.1%).

Compound **1-B3-a:** LCMS (ESI) [M+H]⁺ = 227.2.

Compound **1-B3-a:** ¹H NMR (400 MHz, DMSO-d₆) δ 7.61 (dd, *J =* 8.2, 1.8 Hz, 1H), 7.43 (d, *J =* 1.7 Hz, 1H), 7.03 (d, *J =* 8.2 Hz, 1H), 6.13 (s, 2H), 3.69 (t, *J =* 6.7 Hz, 2H), 3.09 (t, *J =* 7.1 Hz, 2H), 2.05 (p, *J =* 6.9 Hz, 2H).

### Example 4: Preparation of 4-chloro-1-(6-nitrobenzo[d][1,3]dioxol-5-yl)butanone (compound 1-B4-a)

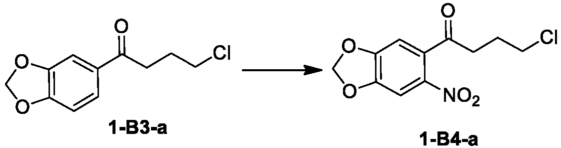

Method 1: Concentrated nitric acid (800 mL) was slowly added to a 2 L reaction flask and cooled to -5°C. Compound **1-B3-a** (80 g, 0.353 mol) was weighed and added to the reaction flask in batches. The reaction mixture was stirred at -5°C for 4 hours, and the reaction was monitored by TLC (PE: EA= 5:1). The reaction mixture was slowly added to 2 L of ice water to precipitate a large amount of solid. After filtration, the filter cake was slurried twice with saturated brine (2 L * 2). After filtration, the filter cake was slurried twice with saturated sodium bicarbonate aqueous solution (1 L * 2), and then slurried once with 2L of water. After filtration, the filter cake was dissolved in ethyl acetate (1 L), dried over anhydrous sodium sulfate, filtered, and concentrated and evaporated to dryness to obtain target compound **1-B4-a** (81 g, yield: 76%, purity: 89.3%).

Method 2: Concentrated nitric acid (12 mL) was slowly added to a reaction flask and cooled to -5°C. Compound **1-B3-a** (4.0 g, 17.6 mmol) was weighed and dissolved in acetic acid (12 mL), and slowly added dropwise to the reaction flask. The reaction mixture was stirred at -5°C for 4 hours, and the reaction was monitored by TLC (PE: EA = 5:1). The reaction mixture was slowly added to 100 mL of ice water to precipitate a large amount of solid. After filtration, the filter cake was slurried twice with saturated brine (100 mL * 2). After filtration, the filter cake was slurried twice with saturated sodium bicarbonate aqueous solution (100 mL * 2), and then slurried once with 100 mL of water. After filtration, the filter cake was dissolved in ethyl acetate (100 mL), dried over anhydrous sodium sulfate, filtered, and concentrated and evaporated to dryness to obtain target compound **1-B4-a** (4 g, yield: 75.7%, purity: 90.7%).

Compound **1-B4-a:** ¹H-NMR (400 MHz, DMSO-d₆) δ 7.72 (s, 1H), 7.27 (s, 1H), 6.30 (s, 2H), 3.70 (t, *J =* 6.7 Hz, 2H), 2.95 (t, *J =* 7.1 Hz, 2H), 2.19 - 1.93 (m, 2H).

### Example 5: Preparation of 1-(6-aminobenzo[d][1,3]dioxol-5-yl)-4-chlorobutan-1-one (compound 1-B5-a)

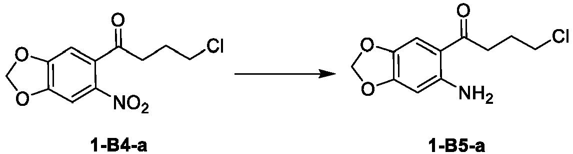

Method 1: Compound **1-B4-a** (250 g, purity: 89.3%, 0.92 mol) was added to ethyl acetate (3 L), and then platinum dioxide (12.5 g) was added thereto. The reaction mixture was replaced with hydrogen three times, and then reacted overnight at room temperature under hydrogen atmosphere. The reaction was monitored by LCMS. After the reaction was completed, the reaction mixture was filtered through diatomite, and the filtrate was concentrated to obtain a crude product of target compound **1-B5-a** (200 g), which was directly used in the next step of conversion.

Method 2: Compound **1-B4-a** (5 g, purity: 89.3%, 0.016 mol) was added to tetrahydrofuran (50 mL), and then platinum dioxide (0.2 g) was added thereto. The reaction mixture was replaced with hydrogen three times, and then reacted overnight at room temperature under hydrogen atmosphere. The reaction was monitored by LCMS. After the reaction was completed, the reaction mixture was filtered through diatomite, and the filtrate was concentrated to obtain a crude product of target compound **1-B5-a** (4 g), which can be directly used in the next step of conversion.

Compound **1-BS-a:** LCMS (ESI) [M+H]⁺ = 242.0.

Compound **1-B5-a:** ¹H NMR (400 MHz, CDCl₃) δ 7.15 (s, 1H), 6.27 (s, 1H), 5.93 (s, 2H), 3.66 (t, *J =* 6.3 Hz, 2H), 3.02 (t, *J =* 7.0 Hz, 2H), 2.18 (p, *J =* 6.7 Hz, 2H).

### Example 6: Preparation of 1-(6-aminobenzo[d][1,3]dioxol-5-yl)-4-hydroxybutan-1-one (compound 1)

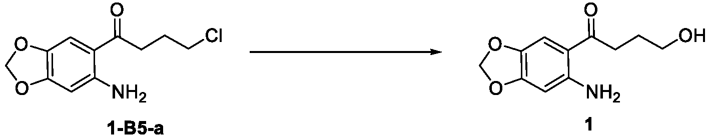

Method 1: Compound **1-B5-a** (200.0 g) was dissolved in acetonitrile (1 L), then water (1 L) was added thereto, and potassium carbonate (137.3 g, 0.994 mol) was added thereto. The reaction mixture was stirred and reacted at 50°C for 8 hours. The reaction was monitored by LCMS. After the reaction was completed, the reaction mixture was concentrated to remove acetonitrile, and the residue was extracted with ethyl acetate (6 L). The organic phase was washed twice with pure water (2 L) and then once with saturated brine (2 L). The organic phase was dried over anhydrous Na₂SO₄ and concentrated to obtain a crude product. The crude product was slurried with ethyl acetate (400 mL) for 1.5 hours. After filtration, the filter cake was slurried with ethyl acetate (250 mL) for 2 hours. After filtration, the filter cake was dried under reduced pressure to obtain compound **1** (112 g, two-step yield calculated from **1-B4-a:** 54%, purity: 97.7%).

Method 2: Compound **1-B5-a** (4.0 g) was dissolved in acetonitrile (20 mL), then water (20 mL) was added thereto, and potassium hydroxide (1.12 g, 0.02 mol) was added thereto. The reaction mixture was stirred and reacted at 50°C for 8 hours. The reaction was monitored by LCMS. After the reaction was completed, the reaction mixture was concentrated to remove acetonitrile, and the residue was extracted with ethyl acetate (120 mL). The organic phase was washed twice with pure water (120 mL) and then once with saturated brine (120 mL). The organic phase was dried over anhydrous Na₂SO₄ and concentrated to obtain a crude product. The crude product was slurried with ethyl acetate (8 mL) for 1.5 hours. After filtration, the filter cake was slurried with ethyl acetate (5 mL) for 2 hours. After filtration, the filter cake was dried under reduced pressure to obtain compound **1** (2.2 g, two-step yield calculated from **1-B4-a:** 53%, purity: 98.0%).

Compound **1:** LCMS (ESI) [M-18+H]⁺ = 206.2.

Compound **1:** ¹H NMR (400 MHz, CD₃OD) δ 7.18 (s, 1H), 6.24 (s, 1H), 5.87 (s, 2H), 3.62 (t, *J =* 6.7 Hz, 2H), 2.95 - 2.84 (m, 2H), 1.95 - 1.82 (m, 2H).

### Example 7: Preparation of (5)-7-ethyl-7-hydroxy-14-(3-hydroxypropyl)-10,13-dihydro-11H-[1,3]dioxolo[4,5-g]pyrano[3',4':6,7]indolizino[1,2-b]quinoline-8,11(7H)-dione (compound 3)

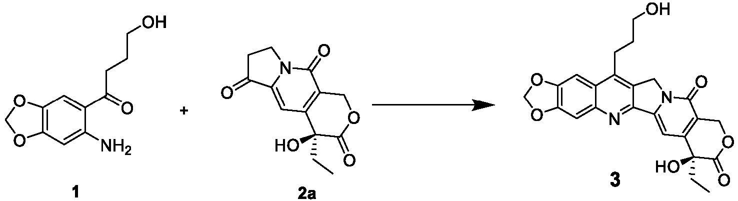

Method 1: Compound **1** (41.80 g, 0.19 mol), compound **2a** (110.00 g, 0.42 mol), and *p*-toluenesulfonic acid monohydrate (31.90 g, 0.17 mol) were added to a three-necked flask. 275 mL of NMP was weighed and added to the flask, and the reaction mixture was heated and stirred at 110°C under nitrogen atmosphere. Compound **1** (31.35 g, 0.14 mol) was added to the reaction system every 0.5 hours for a total of 3 times. After the addition was completed, the reaction mixture was stirred for another 1 hour, and the reaction was monitored by HPLC. After the reaction was completed, the reaction mixture was added with isopropanol (550 mL) and stirred for 1.0 hour. The resulting suspension was added to methyl *tert*-butyl ether (11 L) and stirred for 1 hour. After filtration under reduced pressure, the filter cake was slurried with water (5.5 L) for 1 hour. The resulting filter cake was subjected to forced air drying at 50°C for 16 hours, and then dried under reduced pressure at 80°C for 4 hours to obtain compound **3** (182.00 g, yield: 77%, purity: 80.2%).

Method 2: Compound **1** (41.80 g, 0.19 mol), compound **2a** (110.00 g, 0.42 mol), and p-toluenesulfonic acid monohydrate (31.90 g, 0.17 mol) were added to a three-necked flask. 550 mL of NMP was weighed and added to the flask, and the reaction mixture was heated and stirred at 110°C under nitrogen atmosphere. Compound **1** (31.35 g, 0.14 mol) was added to the reaction system every 0.5 hours for a total of 3 times. After the addition was completed, the reaction mixture was stirred for another 1 hour. The reaction mixture was added with isopropanol (110 mL), then added dropwise with water (1.32 L), cooled to 5°C, and stirred for 1.0 hour. After filtration under reduced pressure, the filter cake was washed with water, and slurried with water (13.75 L) for 2 hours. After filtration under reduced pressure, the filter cake was again washed with water. The filter cake was added to the reaction flask, then DMF (165 mL) and methanol (1.32 L) were sequentially added thereto, and the reaction mixture was stirred at 25°C for 12 hours. After filtration under reduced pressure, the filter cake was washed with methanol, and dried under vacuum at 50°C for 48 hours to obtain compound **3** (184.0 g, yield: 77.9%, purity: 93%).

Comparative example 1: Compound **1** (1.23 g, 5.5 mmol), compound **2a** (1.0 g, 3.8 mmol), and *p*-toluenesulfonic acid monohydrate (289 mg, 1.5 mmol) were added to a three-necked flask. 2.5 mL of NMP was weighed and added to the flask, and the reaction mixture was heated and stirred at 110°C for 2 hours under nitrogen atmosphere. The reaction mixture was added with isopropanol (1.0 mL), then added dropwise with water (20 mL), cooled to 5°C, and stirred for 1.0 hour. After filtration under reduced pressure, the filter cake was washed with water, and slurried with water (20 mL) for 2 hours. After filtration under reduced pressure, the filter cake was again washed with water. The filter cake was added to the reaction flask, then DMF (1.5 mL) and methanol (12 mL) were sequentially added thereto, and the reaction mixture was stirred at 25°C for 12 hours. After filtration under reduced pressure, the filter cake was washed with methanol, and dried under vacuum at 50°C for 48 hours to obtain compound 3 (0.68 g, yield: 39.7%, purity: 91%).

Comparative example 2: Compound **1** (0.39 g, 1.7 mmol), compound **2a** (1.0 g, 3.8 mmol), and p-toluenesulfonic acid monohydrate (289 mg, 1.5 mmol) were added to a three-necked flask. 2.5 mL of NMP was weighed and added to the flask, and the reaction mixture was heated and stirred at 65°C under nitrogen atmosphere. Compound **1** (0.28 g, 1.26 mmol) was added to the reaction system every 0.5 hours for a total of 3 times. After the addition was completed, the reaction mixture was stirred for another 1 hour, and the reaction was monitored by HPLC to show the presence of a large number of uncyclized intermediates and few products.

Compound **3:** LCMS (ESI) [M+H]⁺ = 451.4.

Compound **3:** ¹H NMR (400 MHz, DMSO-d₆) δ 7.59 (s, 1H), 7.49 (s, 1H), 7.25 (s, 1H), 6.49 (s, 1H), 6.30 (s, 2H), 5.54 - 5.36 (m, 2H), 5.19 (s, 2H), 4.68 (t, *J=* 5.0 Hz, 1H), 3.51 (q, *J =* 5.6 Hz, 2H), 3.14 (t, *J =* 7.6 Hz, 2H), 1.99 - 1.73 (m, 4H), 0.92 (t, *J =* 7.3 Hz, 3H).

### Example 8: Preparation of (5)-7-ethyl-7-hydroxy-14-(3-hydroxypropyl)-10,13-dihydro-11H-[1,3]dioxolo[4,5-g]pyrano[3',4':6,7]indolizino[1,2-b]quinoline-8,11(7H)-dione (compound 3)

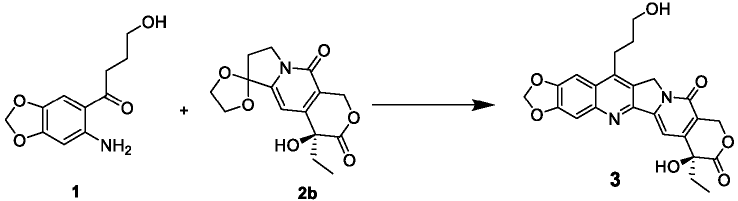

Compound **1** (0.076 g, 0.34 mmol), compound **2b** (0.2 g, 0.65 mmol), and *p-*toluenesulfonic acid monohydrate (0.05 g, 0.26 mmol) were added to a 25 mL three-necked flask. NMP (0.50 mL) was added thereto, then magnets were added thereto, and the reaction mixture was stirred at 110°C under argon atmosphere. Compound **1** was added thereto every 0.5 hours at an amount of (0.057 g, 0.26 mmol), (0.057 g, 0.26 mmol), and (0.038 g, 0.17 mmol). After the addition was completed, the reaction mixture was stirred for another 0.5 hours. Samples were taken and detected by LCMS. After the reaction was completed, the reaction mixture was added with 2 mL of isopropanol under stirring, and stirred for 1.0 hour. The resulting suspension was added with 40 mL of methyl *tert*-butyl ether and stirred for 0.5 hours. After filtration under reduced pressure, the filter cake was slurried with 40 mL of water for 0.5 hours. The resulting filter cake was subjected to forced air drying at 50°C for 12 hours to obtain a crude product of compound **3** (0.20 g, yield: 56%, purity: 80%).

Compound **3:** LCMS (ESI) [M+H]⁺ = 451.4.

Compound **3:** ¹H NMR (400 MHz, DMSO-d₆) δ 7.59 (s, 1H), 7.49 (s, 1H), 7.25 (s, 1H), 6.49 (s, 1H), 6.30 (s, 2H), 5.54 - 5.36 (m, 2H), 5.19 (s, 2H), 4.68 (t, *J=* 5.0 Hz, 1H), 3.51 (q, *J =* 5.6 Hz, 2H), 3.14 (t, *J =* 7.6 Hz, 2H), 1.99 - 1.73 (m, 4H), 0.92 (t, *J =* 7.3 Hz, 3H).

### Example 9: Preparation of (9H-fluoren-9-yl)methyl (5)-(2-(((3-(7-ethyl-7-hydroxy-8,11-dioxo-7,8,11,13-tetrahydro-10H-[1,3]dioxolo[4,5-g] pyrano [3',4':6,7] indolizino [1,2-b] quinolin-14-yl)propoxy)methyl)amino)-2-oxoethyl)carbamate (compound 5a)

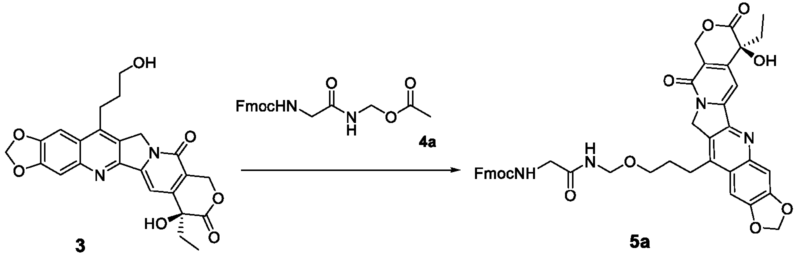

Method 1: DMF (1.75 L) was added to a reaction flask at room temperature. Compound **3** (175.66 g, purity: 80.2%, 313 mmol) and compound **4a** (345.93 g, 939 mmol) were then sequentially added thereto. The reaction mixture was replaced with argon three times, and HCl/EA (2 N, 94 mL, 187.8 mmol) was added dropwise thereto at room temperature. The reaction was monitored by HPLC.

After the reaction was completed, the reaction mixture was added dropwise to water (17.5 L) at room temperature. After the dropwise addition was completed, the reaction mixture was stirred for 1 hour. After filtration under reduced pressure, the filter cake was added with water (8.75 L) and stirred for 1 hour. After filtration under reduced pressure, the filter cake was dissolved in DCM (3.5 L) under stirring. The resulting DCM system was washed with water (3.5 L * 2). The organic phase was separated and dried over anhydrous sodium sulfate (875.00 g). The organic phase was filtered and concentrated to obtain a crude product. The crude product was purified by silica gel column chromatography (eluent: DCM/THF/EA/Acetone) to obtain compound **5a** (198.44 g, yield: 70%, purity: 84%).

Method 2: DMF (20 mL) was added to a reaction flask at 30°C. Compound **3** (2.0 g, purity: 80.2%, 3.56 mmol) and compound **4a** (3.94 g, 10.7 mmol) were then sequentially added thereto. The reaction mixture was replaced with argon three times, and BF₃·Et₂O (1.01 g, 7.12 mmol) was added thereto at room temperature.

After the reaction was completed, the reaction mixture was added dropwise to water (200 mL) at room temperature. After the dropwise addition was completed, the reaction mixture was stirred for 1 hour. After filtration under reduced pressure, the filter cake was added with water (100 mL) and stirred for 1 hour. After filtration under reduced pressure, the filter cake was dissolved in DCM (40 mL) under stirring. The resulting DCM system was washed with water (40 mL * 2). The organic phase was separated and dried over anhydrous sodium sulfate. The organic phase was filtered and concentrated to obtain a crude product. The crude product was purified by silica gel column chromatography (eluent: DCM/THF/EA/Acetone) to obtain compound **5a** (2.72 g, yield: 67.8%, purity: 84%).

Compound **5a:** LCMS (ESI) [M+H]⁺ = 759.5.

Compound **5a:** ¹H NMR (400 MHz, DMSO-d₆) δ 8.65 (t, *J =* 6.4 Hz, 1H), 7.84 (d, J = 7.6 Hz, 2H), 7.68 (d, *J =* 7.4 Hz, 2H), 7.56 - 7.50 (m, 2H), 7.45 (s, 1H), 7.38 (t, *J =* 7.3 Hz, 2H), 7.29 (t, *J =* 7.3 Hz, 2H), 7.22 (s, 1H), 6.45 (s, 1H), 6.25 (s, 2H), 5.47 - 5.34 (m, 2H), 5.20 - 5.03 (m, 2H), 4.60 (d, *J =* 6.5 Hz, 2H), 4.27 (d, *J =* 6.8 Hz, 2H), 4.22 - 4.16 (m, 1H), 3.66 (d, *J =* 6.0 Hz, 2H), 3.50 (t, *J =* 6.0 Hz, 2H), 3.13 - 2.95 (m, 2H), 1.94 - 1.77 (m, 4H), 0.88 (t, *J* = 7.3 Hz, 3H).

### Example 10: Preparation of (S)-2-amino-N-((3-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propoxy)methyl)acetamide (compound 6a)

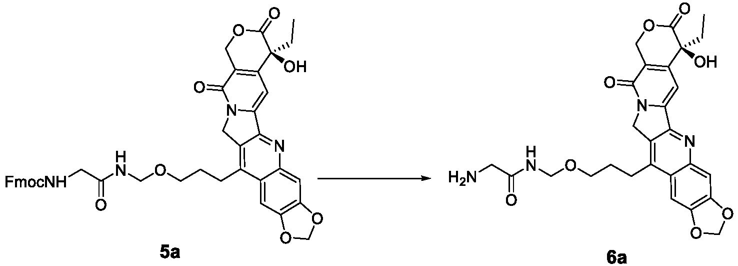

Method 1: Compound **5a** (33.30 g, purity: 84%, 36.9 mmol) and DMF (333 mL) were added to a reaction flask at room temperature and stirred to dissolve, and the above mixture was added dropwise to a solution of diethylamine (4.86 g, 66.42 mmol) in DMF (333 mL). The above reaction mixture was stirred at room temperature for 1 hour, and added dropwise to a mixed solvent of methyl *tert*-butyl ether (7 L) and n-heptane (7 L) which had been pre-cooled in an ice bath. After the dropwise addition was completed, the reaction mixture was stirred for another 2 hours. After filtration under reduced pressure, the filtered solid was dried under reduced pressure to obtain compound **6a** (22.53 g, yield: 88%, purity: 77.7%).

Method 2: Compound **5a** (2.0 g, purity: 84%, 2.2 mmol) and DMF (20 mL) were added to a reaction flask at room temperature and stirred to dissolve, and the above mixture was added dropwise to a solution of piperidine (0.34 g, 4.0 mmol) in DMF (20 mL). The above reaction mixture was stirred at room temperature for 1 hour, and added dropwise to a mixed solvent of methyl *tert*-butyl ether (40 mL) and *n*-heptane (40 mL) which had been pre-cooled in an ice bath. After the dropwise addition was completed, the reaction mixture was stirred for another 2 hours. After filtration under reduced pressure, the filtered solid was dried under reduced pressure to obtain compound **6a** (1.3 g, yield: 82.6%, purity: 75.0%).

Compound **6a:** LCMS (ESI) [M+H]⁺ = 537.4.

The NMR data for formate of compound **6a** are as follows:

¹H NMR (400 MHz, DMSO-d₆) δ 8.78 (t, *J =* 6.6 Hz, 1H), 8.23 (s, 1H), 7.60 (s, 1H), 7.51 (s, 1H), 7.25 (s, 1H), 6.49 (s, 1H), 6.29 (s, 2H), 5.43 (s, 2H), 5.22 (s, 2H), 4.62 (d, *J=* 6.5 Hz, 2H), 3.53 - 3.46 (m, 2H), 3.29 (s, 2H), 3.15 - 3.06 (m, 2H), 1.98 - 1.75 (m, 4H), 0.88 (t, *J* = 7.3 Hz, 3H).

### Example 11: Preparation of N²-((9H-fluoren-9-yl)methoxy)carbonyl)-L-valyl)-N⁶-(tert-butoxycarbonyl)-L-lysine (compound 7-A3-a)

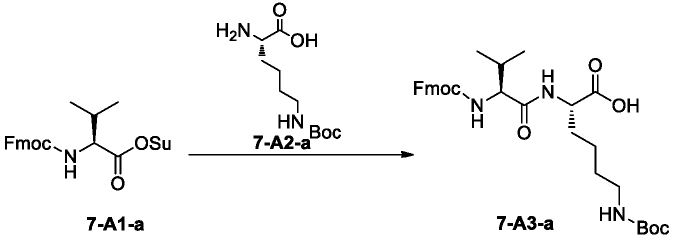

Method 1: Compound **7-A1-a** (100 g, 0.229 mol) and compound **7-A2-a** (56.4 g, 0.229 mol) were placed in a reaction flask, then acetone/water with a ratio of 1:1 (v:v, 1 L) was added thereto under stirring, and NaHCO₃ (76.95 g, 0.916 mol) was added thereto. The reaction mixture was stirred overnight at room temperature. The reaction was monitored by LCMS. The solid in the reaction mixture was filtered, and the filtrate was concentrated. The concentrate was added with HCl (4 N) to adjust the pH to approximately 5, and extracted with ethyl acetate (0.75 L * 2). The organic phases were combined, washed with saturated brine (300 mL * 2), dried over anhydrous sodium sulfate, and the filtrate was concentrated under reduced pressure to obtain target compound **7-A3-a** (117 g, yield: 90%, purity: 97%).

Method 2: Compound **7-A1-a** (100 g, 0.229 mol) and compound **7-A2-a** (56.4 g, 0.229 mol) were placed in a reaction flask, then tetrahydrofuran/water with a ratio of 1:1 (v:v, 1 L) was added thereto under stirring, and Na₂CO₃ (97.1 g, 0.916 mol) was added thereto. The reaction mixture was stirred overnight at room temperature. The reaction was monitored by LCMS. The solid in the reaction mixture was filtered, and the filtrate was concentrated. The concentrate was added with HCl (4 N) to adjust the pH to approximately 5, and extracted with ethyl acetate (0.75 L * 2). The organic phases were combined, washed with saturated brine (300 mL * 2), dried over anhydrous sodium sulfate, and the filtrate was concentrated under reduced pressure to obtain target compound **7-A3-a** (118.3 g, yield: 91.0%, purity: 97%).

Compound **7-A3-a:** LCMS (ESI) [M+H]⁺ = 568.3.

Compound **7-A3-a:** ¹H NMR (400 MHz, DMSO-d₆) δ 7.91 (d, *J =* 7.5 Hz, 2H), 7.82 - 7.73 (m, 2H), 7.64 (d, *J =* 6.9 Hz, 1H), 7.58 (d, *J =* 9.1 Hz, 1H), 7.44 (t, *J =* 7.4 Hz, 2H), 7.40 - 7.31 (m, 2H), 6.68 (brs, 1H), 4.38 - 4.21 (m, 3H), 3.99 - 3.91 (m, 1H), 3.87 (dd, *J =* 8.8, 6.8 Hz, 1H), 2.92 - 2.80 (m, 2H), 2.17 - 2.01 (m, 1H), 1.70-1.67 (m, 1H), 1.64 - 1.52 (m, 1H), 1.42 - 1.16 (m, 13H), 0.93 - 0.83 (m, 6H).

### Example 12: Preparation of (((9H-fluoren-9-yl)methoxy)carbonyl)-Z-valyl-Z-lysine hydrochloride (hydrochloride of compound 7-A4-a)

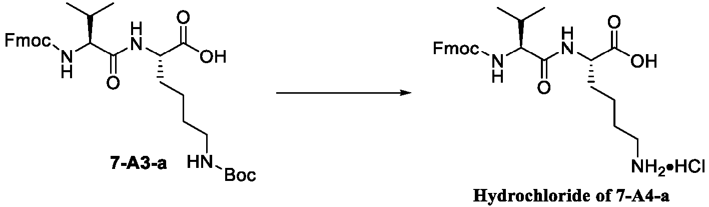

Compound **7-A3-a** (117 g, 206.2 mmol) was added with dioxane (1.5 L) and stirred for 10 minutes. HCl (0.75 L, 4 N in dioxane) was then added thereto, and the reaction mixture was reacted overnight at room temperature. The reaction was monitored by LCMS. The reaction mixture was added with methyl *tert*-butyl ether (2 L), stirred for 30 minutes, and filtered. The solid was transferred to a forced air drying oven for drying to obtain the **hydrochloride of** target compound **7-A4-a** (94 g, yield: 91%, purity: 94%).

LCMS (ESI) [M+H]⁺ = 468.3.

¹H NMR (400 MHz, DMSO-d₆) δ 8.24 (d, *J =* 7.3 Hz, 1H), 8.07 (brs, 3H), 7.90 (d, *J* = 7.5 Hz, 2H), 7.77 (dd, *J =* 7.1, 3.4 Hz, 2H), 7.47 - 7.37 (m, 3H), 7.34 (td, *J =* 7.4, 1.7 Hz, 2H), 4.34 - 4.17 (m, 4H), 3.96 (dd, *J =* 8.6, 7.4 Hz, 1H), 2.83 - 2.65 (m, 2H), 2.04 (dd, *J =* 13.5, 6.7 Hz, 1H), 1.82 - 1.52 (m, 4H), 1.51 - 1.30 (m, 2H), 0.96 - 0.84 (m, 6H).

### Example 13: Preparation of N²-((((9H-fluoren-9-yl)methoxy)carbonyl)-L-valyl)-N⁶,N⁶-dipropyl-L-lysine (compound 7a)

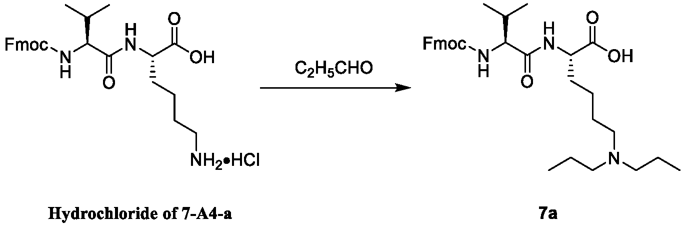

Method 1: The **hydrochloride of** compound **7-A4-a** (55.0 g, 109.1 mmol) and acetic acid (10 mL) were dissolved in a solution of methanol (500 mL). *n*-Propionaldehyde (25.35 g, 436.4 mmol) was added thereto in an ice-water bath, and the reaction mixture was stirred at room temperature for 30 minutes. Sodium cyanoborohydride (27.43 g, 436.4 mmol) was then added thereto in an ice-water bath, and the reaction mixture was stirred at room temperature for 1 hour. LCMS showed the starting material was not completely reacted. *n-*Propionaldehyde (12.67 g, 218.2 mmol) and sodium cyanoborohydride (13.72 g, 218.2 mmol) were added thereto, and the reaction mixture was stirred for another 0.5 hours. The reaction mixture was filtered, and the filtrate was concentrated. The concentrate was added with purified water (300 mL) and extracted with DCM (300 mL * 2). The organic phase was washed with saturated brine, dried over anhydrous Na₂SO₄, and concentrated to obtain a crude product. The crude product was purified by column chromatography (eluent: MeOH and DCM) to obtain compound **7a** (39 g, yield: 65%, purity: 98%).

Method 2: The **hydrochloride of** compound **7-A4-a** (55.0 g, 109.1 mmol) and acetic acid (10 mL) were dissolved in a solution of methanol (500 mL). Sodium cyanoborohydride (27.43 g, 436.4 mmol) was added thereto in an ice-water bath, and the reaction mixture was stirred at room temperature for 30 minutes. *n*-Propionaldehyde (25.35 g, 436.4 mmol) was then added thereto in an ice-water bath, and the reaction mixture was stirred at room temperature for 1 hour. The reaction mixture was filtered, and the filtrate was concentrated. The concentrate was added with water (300 mL) and extracted with DCM (300 mL * 2). The organic phase was washed with saturated brine, dried over anhydrous Na₂SO₄, and concentrated to obtain a crude product. The crude product was dissolved in 110 mL of DCM and added dropwise to 1.1 L of methyl *tert*-butyl ether. The reaction mixture was stirred for 30 minutes and filtered under reduced pressure to obtain compound **7a** (48 g, yield: 80%, purity: 93%).

Compound **7a:** LCMS (ESI) [M+H]⁺ = 552.3.

Compound **7a:** ¹H NMR (400 MHz, DMSO-d₆) δ 7.95 - 7.85 (m, 3H), 7.76 (t, *J =* 7.3 Hz, 2H), 7.51 - 7.40 (m, 3H), 7.35 (t, *J =* 7.4 Hz, 2H), 4.34 - 4.22 (m, 3H), 4.13 (dd, *J =* 12.6, 7.2 Hz, 1H), 3.91 (dd, *J =* 8.8, 7.1 Hz, 1H), 2.52 - 2.41 (m, 6H), 2.03 (dt, *J =* 13.5, 6.7 Hz, 1H), 1.79 - 1.67 (m, 1H), 1.66 - 1.56 (m, 1H), 1.42 - 1.36 (m, 6H), 1.36 - 1.21 (m, 2H), 0.90 (t, *J* = 6.9 Hz, 6H), 0.82 (t, *J =* 6.9 Hz, 6H).

### Example 14: Preparation of (9H-fluoren-9-yl)methyl ((11S,14S)-11-(4-(dipropylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecan-14-yl)carbamate (compound 8a)

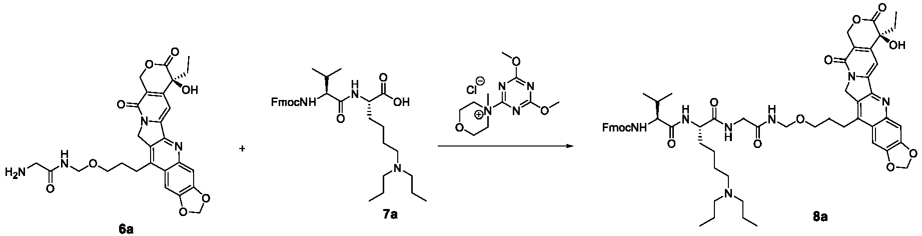

Method 1: Compound **6a** (19.88 g, purity: 77.7%, 28.79 mmol), compound **7a** (19.06 g, 34.55 mmol), and DMF (500 mL) were sequentially added to a reaction flask at room temperature. The reaction mixture was stirred at room temperature for 15 minutes, cooled to 0°C under argon atmosphere, and 4-(4,6-dimethoxytriazin-2-yl)-4-methylmorpholinium chloride monohydrate (10.17 g, 34.55 mmol) was added thereto. The reaction mixture was cooled to 0°C under argon atmosphere, and after the addition was completed, the reaction was continued at 0°C. The reaction was monitored by LCMS.

After the reaction was completed, the reaction mixture was added dropwise with methyl *tert*-butyl ether (10 L), stirred for 1.5 hours, and filtered. The filtered solid was dissolved in 600 mL of a mixed solvent of DCM/MeOH (DCM: MeOH = 10:1), and the resulting mixture was concentrated under reduced pressure to obtain a crude product. The crude product was purified by silica gel column chromatography (eluent: DCM: MeOH) to obtain compound **8a** (24.00 g, yield: 74%, purity: 95.1%).

Method 2: Compound **6a** (2.0 g, purity: 77.7%, 2.9 mmol), compound **7a** (2.08 g, 3.77 mmol), and DMF (50 mL) were sequentially added to a reaction flask at room temperature. The reaction mixture was stirred at room temperature for 15 minutes, cooled to -5°C, and 4-(4,6-dimethoxytriazin-2-yl)-4-methylmorpholinium chloride monohydrate (1.11 g, 3.77 mmol) was added thereto. The reaction mixture was cooled to -5°C under argon atmosphere, and after the addition was completed, the reaction was continued at -5°C. The reaction was monitored by LCMS.

After the reaction was completed, the reaction mixture was added dropwise with methyl *tert*-butyl ether (1 L), stirred for 1.5 hours, and filtered. The filtered solid was dissolved in 60 mL of a mixed solvent of DCM/MeOH (DCM: MeOH = 10:1), and the resulting mixture was concentrated under reduced pressure to obtain a crude product. The crude product was purified by silica gel column chromatography (eluent: DCM: MeOH) to obtain compound **8a** (2.35 g, yield: 71.9%, purity: 95.0%).

Compound **8a:** LCMS (ESI) [M+H]⁺ = 1070.7.

Compound **8a:** ¹H NMR (400 MHz, DMSO-d₆) δ 8.67 (t, *J* = 6.6 Hz, 1H), 8.28 (t, *J=* 5.9 Hz, 1H), 8.09 (d, *J =* 7.4 Hz, 1H), 7.88 (d, *J =* 7.5 Hz, 2H), 7.77 - 7.70 (m, 2H), 7.57 (s, 1H), 7.53 - 7.37 (m, 4H), 7.36 - 7.28 (m, 2H), 7.26 (s, 1H), 6.50 (s, 1H), 6.30 (s, 2H), 5.77 (s, 1H), 5.44 (d, *J =* 2.5 Hz, 2H), 5.20 (s, 2H), 4.67 - 4.53 (m, 2H), 4.36 - 4.17 (m, 4H), 3.93 - 3.85 (m, 1H), 3.84 - 3.70 (m, 2H), 3.52 (t, *J =* 6.1 Hz, 2H), 3.16 - 3.04 (m, 2H), 3.02 - 2.80 (m, 5H), 2.01 - 1.50 (m, 13H), 1.38 - 1.28 (m, 2H), 0.95 - 0.80 (m, 15H).

### Example 15: Preparation of (S)-2-((S)-2-amino-3-methylbutanamido)-6-(dipropylamino)-N-(2-(((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propoxy)methyl)amino)-2-oxoethyl)hexanamide (compound 9a)

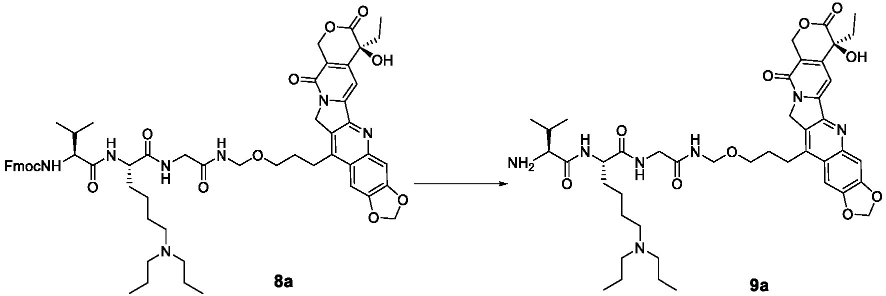

Method 1: Compound **8a** (29.50 g, 27.56 mmol) and DMF (147.5 mL) were added to a reaction flask in an ice bath under argon atmosphere, then the stirring was started, and diethylamine (7.4 mL) was added dropwise thereto. After the dropwise addition was completed, the ice bath was removed, and the reaction was continued and monitored by LCMS. The reaction mixture was added with tetrahydrofuran (295 mL) and concentrated under reduced pressure. The operation was repeated three times. A solution of compound **9a** in DMF was obtained, which was directly used in the next reaction step (conversion rate was calculated as 100%).

Method 2: Compound **8a** (2.0 g, 1.87 mmol) and THF (20 mL) were added to a reaction flask, then the stirring was started, and *N,N*-diisopropylethylamine (2 mL) was added dropwise thereto. After the dropwise addition was completed, the reaction mixture was reacted at room temperature for 6 hours. A solution of compound **9a** in THF was obtained, which was directly used in the next reaction step (conversion rate was calculated as 100%).

Compound **9a:** LCMS (ESI) [M+H]⁺ = 848.6.

Compound **9a:** ¹H NMR (400 MHz, DMSO-d₆, trifluoroacetate of compound **9a**) δ 9.22 (s, 1H), 8.68 (t, *J =* 6.5 Hz, 1H), 8.54 (d, *J =* 7.6 Hz, 1H), 8.36 (t, *J =* 5.8 Hz, 1H), 8.08 (d, *J =* 3.6 Hz, 3H), 7.59 (s, 1H), 7.51 (s, 1H), 7.25 (s, 1H), 6.50 (brs, 1H), 6.29 (d, *J =* 1.1 Hz, 2H), 5.42 (s, 2H), 5.23 (s, 2H), 4.67 - 4.53 (m, 2H), 4.35 (q, *J =* 7.3 Hz, 1H), 3.83 - 3.69 (m, 2H), 3.66 - 3.61 (m, 1H), 3.15 - 3.06 (m, 2H), 3.04 - 2.94 (m, 6H), 2.10 - 1.95 (m, 1H), 1.93 - 1.78 (m, 4H), 1.76 - 1.54 (m, 8H), 1.42 - 1.26 (m, 2H), 0.95 - 0.85 (m, 15H).

### Example 16: Preparation of 6-(2-(methylsulfonyl)pyrimidin-5-yl)hex-5-ynoyl chloride (compound 10a)

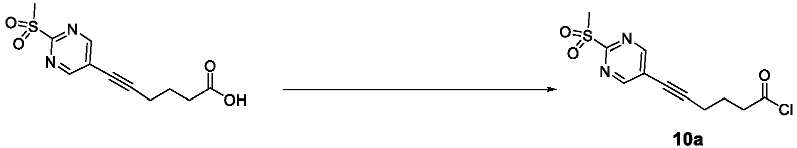

6-(2-(Methylsulfonyl)pyrimidin-5-yl)hex-5-ynoic acid (7.76 g, 28.95 mmol) and DCM (77.6 mL) were added to a reaction flask at room temperature under argon atmosphere, then the stirring was started, and thionyl chloride (6.88 g, 57.83 mmol) was added dropwise thereto. After the dropwise addition was completed, the reaction mixture was reacted for 1 hour. After the reaction was completed, the reaction mixture was concentrated under reduced pressure. The concentrate was added with DCM (77.6 mL) and concentrated under reduced pressure. After the operation was repeated twice, the concentrate was added with DCM (100 mL) to obtain a solution of acid chloride intermediate **10a** in DCM, which was directly used in the next reaction step (conversion rate was calculated as 100%).

Compound **10a** was quenched with methanol to obtain methyl ester: LCMS (ESI) [M+H]⁺ = 283.0.

### Example 17: Preparation of N-((11S,14S)-11-(4-(dipropylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecan-14-yl)-6-(2-(methylsulfonyl)pyrimidin-5-yl)hex-5-ynamide (compound Ia)

### Method 1:

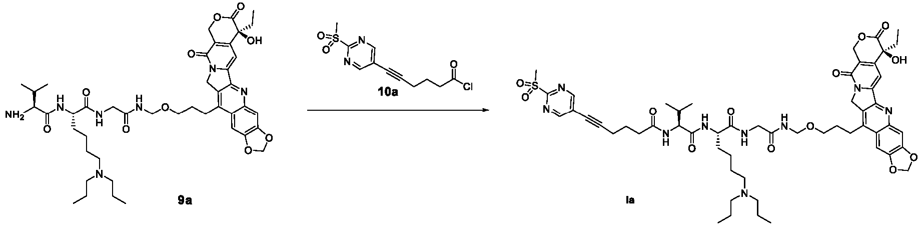

A solution of compound **9a** in DMF (prepared by method 1 in example 15, 23.36 g, 27.56 mmol) and DIPEA (5.34 g, 41.38 mmol) were added to a reaction flask at room temperature under argon atmosphere, and the stirring was started. A solution of compound 10a in DCM (prepared in example 16, 8.30 g, 28.95 mmol) was added dropwise thereto in an ice bath. After the dropwise addition was completed, the reaction was continued in an ice bath and monitored by LCMS. The reaction mixture was diluted with DCM (1.4 L). The organic phase was sequentially washed with 2% sodium bicarbonate aqueous solution (1.4 L * 2) and saturated brine (1.4 L * 2). The organic phase was dried over anhydrous sodium sulfate, filtered, and the filtrate was concentrated under reduced pressure to obtain a crude product. The crude product was purified by preparative HPLC to obtain **formate of** compound **Ia** (18 g, yield calculated from compound 8a: 57%) with the HPLC purity (method I) of 94.4% and the chiral purity of 98.68%.

### Method 2:

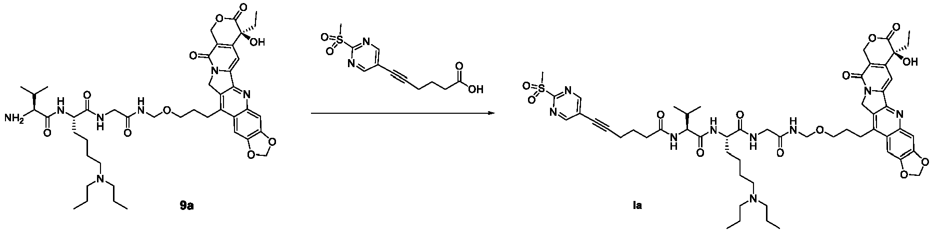

6-(2-(Methylsulfonyl)pyrimidin-5-yl)hex-5-ynoic acid (0.5 g, 1.87 mmol) was weighed and dissolved in DMF (5 mL), and added to a solution of 9a in THF (prepared by method 2 in example 15, 1.58 g, 1.87 mmol). 4-(4,6-Dimethoxytriazin-2-yl)-4-methylmorpholinium chloride monohydrate (0.55 g, 1.87 mmol) was then added thereto in an ice bath. The ice bath was removed and the reaction was carried out at room temperature for 3 hours. The reaction mixture was purified by preparative HPLC to obtain **formate of** compound **Ia** (1.23 g, yield calculated from compound 8a: 60%).

**Formate of** compound **Ia:** LCMS (ESI) [M+H]⁺ = 1098.5.

**Formate of** compound **Ia:** ¹H NMR (400 MHz, DMSO-d₆) δ 9.09 (s, 2H), 8.56 (t, *J* = 6.5 Hz, 1H), 8.17 (s, 1H), 8.14 (t, *J =* 5.7 Hz, 1H), 7.97 (d, *J =* 7.3 Hz, 1H), 7.89 (d, *J =* 8.6 Hz, 1H), 7.58 (s, 1H), 7.50 (s, 1H), 7.24 (s, 1H), 6.45 (s, 1H), 6.28 (s, 2H), 5.48 - 5.35 (m, 2H), 5.24 (s, 2H), 4.64 - 4.54 (m, 2H), 4.23 - 4.07 (m, 2H), 3.79 - 3.66 (m, 2H), 3.50 (t, *J =* 5.9 Hz, 2H), 3.40 (s, 3H), 3.14 - 3.08 (t, *J* = 8.22 Hz, 2H), 2.59 - 2.52 (m, 2H), 2.36 - 2.27 (m, 8H), 2.00 - 1.76 (m, 7H), 1.72 - 1.62 (m, 1H), 1.58 - 1.48 (m, 1H), 1.40 - 1.20 (m, 8H), 0.88 - 0.77 (m, 15H).

### Preparative HPLC chromatographic conditions

Chromatographic column: C18 preparative column (100 mm × 250 mm, 10 µm), wavelength: 214 nm; flow rate: 250 mL/min;
mobile phase A: 0.1% formic acid aqueous solution;
mobile phase B: acetonitrile

### Gradient table:

| | | | | | | | | |
|---|---|---|---|---|---|---|---|---|
| Time (min) | 0 | 8 | 18 | 30 | 35 | 45 | 50 | 55 |
| Mobile phase A (%) | 85 | 85 | 76 | 76 | 70 | 70 | 65 | 60 |
| Mobile phase B (%) | 15 | 15 | 24 | 24 | 30 | 30 | 35 | 40 |

### HPLC chromatographic conditions (method I)

| | | | | | | |
|---|---|---|---|---|---|---|
| Chromatographic conditions | Chromatographic parameters | | | | | |
| Chromatographic column | Waters CORTECS T3, 3.0 × 100 mm 2.7 µm | | | | | |
| Detection wavelength | UV 254 nm | | | | | |
| Injection volume | 5 µL | | | | | |
| Column temperature | 40°C | | | | | |
| Flow rate | 0.8 mL/min | | | | | |
| Elution time | 18 min (gradient elution) | | | | | |
| Diluent | 0.1% trifluoroacetic acid-acetonitrile (60-40) | | | | | |
| Probe wash | Acetonitrile | | | | | |
| Sample concentration | 0.5 mg/mL | | | | | |
| Mobile phase A | 0.1% trifluoroacetic acid | | | | | |
| Mobile phase B | Acetonitrile | | | | | |

| Elution procedure | | | | | | |
|---|---|---|---|---|---|---|
| Time (min) | 0 | 8 | 12 | 15 | 15.1 | 18 |
| Mobile phase A | 80 | 50 | 10 | 10 | 80 | 80 |
| Mobile phase B | 20 | 50 | 90 | 90 | 20 | 20 |

### Chiral HPLC chromatographic conditions

| Name | Parameters |
|---|---|
| Chromatographic column | CHIRALPAK AGP 0.4 cm I.D. × 15 cm L × 5.0 µm |
| Mobile phase | 50 mM ammonium acetate solution (pH 7.0)-isopropanol (96/4) |
| Flow rate | 0.5 mL/min |
| Injection volume | 3 µL |
| Wavelength | 254 nm |
| Column temperature | 20°C |
| Sample solution | 2 mg/mL, dissolved in 10 mM ammonium formate solution (pH 4.5) and mixed well. |

The following drug linker conjugate was synthesized by the above method:

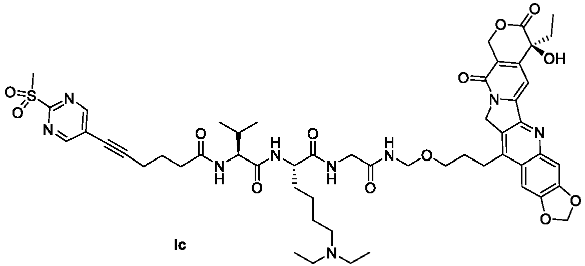

### Example 18: Preparation of antibody 2E3-02

The heavy chain amino acid sequence (SEQ ID NO: 1) of 2E3-02 was subjected to codon optimization and gene synthesis (Sangon Biotech (Shanghai) Co., Ltd.), and then constructed into PTT5 vector, named PPT5-02-CH. The light chain amino acid sequence (SEQ ID NO: 2) of 2E3-02 was subjected to codon optimization and gene synthesis (Sangon Biotech (Shanghai) Co., Ltd.), and then constructed into PTT5 vector, named PTT5-02-CL. Anti-B7H3 antibody expression plasmid PTT5-02-CH/PTT5-02-CL was co-transfected into HEK293F cells (ATCC) for expression by PEI max reagent, and expressed in a 5% COz shaker at 37°C for 7 days. The supernatant was collected and purified by ProA magnetic beads to obtain anti-B7H3 antibody 2E3-02 (whose heavy chain sequence is SEQ ID NO: 1, and whose light chain sequence is SEQ ID NO: 2).

### Example 19: Preparation of ADC

25 mL of antibody 2E3-02 (anti-B7H3 antibody, concentration of 28.5 mg/mL, 20 mM acetate buffer) was taken. 25 mL of 20 mM acetate buffer was added thereto for dilution, and then 1 mL of 0.25 M EDTA aqueous solution was added thereto and mixed homogeneously. The sample was added with 0.5 M disodium hydrogen phosphate aqueous solution to adjust the pH to 7.6, and then 4.5-fold equivalents (relative to the antibody) of 20 mM TCEP (tris(2-carboxyethyl)phosphine hydrochloride) solution was added thereto and mixed homogeneously. The mixture was reacted at room temperature for 90 minutes. Finally, 10-fold equivalents (relative to the antibody) of Ia dissolved in DMSO was added thereto, and the reaction was continued for 2 h at room temperature after mixing homogeneously. After the reaction was completed, the sample was transferred into a 10 mM histidine buffer with a pH of 5.5 using a 30 KDa ultrafiltration tube, and low molecular weight substances were removed. Finally, the sample was concentrated to obtain a solution containing the anti-B7H3 antibody ADC composition (ADC-01), whose DAR value was measured to be 7.98 by mass spectrometry.

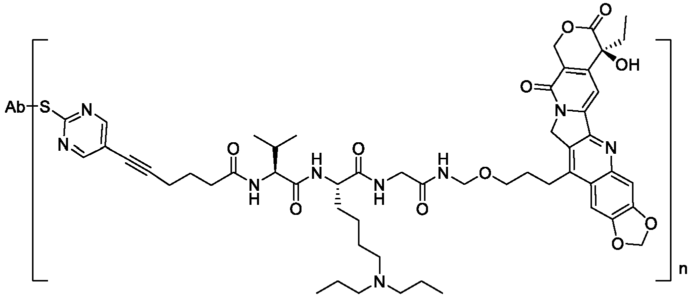

Among them, Ab is B7H3 antibody 2E3-02.

Determination of DAR values as follows:
Chromatographic conditions:
Chromatographic column: PLRP-S, 2.1 * 50 mm, 5 µm;
Mobile phase A: 0.1% FA/H₂O; mobile phase B: 0.1% FA/ACN

Column temperature: 30°C; sample chamber temperature: 8°C; flow rate: 0.6 mL/min; injection volume: 2 µL

| | | | | | |
|---|---|---|---|---|---|
| Time (min) | 1 | 5 | 5.1 | 7 | 10 |
| Mobile phase A | 90 | 40 | 10 | 90 | 90 |
| Mobile phase B | 10 | 60 | 90 | 10 | 10 |

Sample treatment: 50 µg of sample ADC-01 was taken and 2 µL of 1 M DTT was added thereto. The mixture was diluted with 50 µL of ultrapure water to a concentration of approximately 1.0 mg/mL, mixed homogeneously, and reduced at room temperature for 30 minutes.

LC/MS model: Agilent 1290-6545XT Q-TOF.

Mass spectrometry conditions: Gas temp: 320°C, drying gas: nebulizer: 35 psi; sheath gas temp: 350°C; sheath gas flow: 11 L/min; m/z 500 to 3000.

The results are as follows:

| Peptide chain | | mAb | DAR1 | DAR2 | DAR3 |
|---|---|---|---|---|---|
| LC | Theoretical value | 23245 | 24262.4 | 25279.8 | 26297.2 |
| | Experimental value | 23247.41 | 24265.49 | Not detected | Not detected |
| HC | Theoretical value | 50367 | 51384.4 | 52401.8 | 53419.2 |
| | Experimental value | Not detected | Not detected | Not detected | 53426.64 |

In the table, mAb represents an unconjugated antibody; LC represents a light chain of an antibody; HC represents a heavy chain of an antibody; DAR1 represents a conjugate containing a light or heavy chain conjugated with one toxin molecule; DAR2 represents a conjugate containing a light or heavy chain conjugated with two toxin molecules; DAR3 represents a conjugate containing a light or heavy chain conjugated with three toxin molecule; wherein the theoretical molecular weight of the monoclonal antibody is calculated based on the G0F glycoform. Hereinafter, mAb, LC, HC, DAR1, DAR2, and DAR3 are described as above.

It was determined that the light chain of antibody 2E3-02 was conjugated with 0 to 1 toxin molecule (LC and DAR1 percentages were 1.0% and 99.0%, respectively), while the heavy chain was conjugated with 0 to 3 toxin molecules (mAb, DAR1, DAR2, and DAR3 percentages were 0%, 0%, 0%, and 100%, respectively), and thus the drug-to-antibody ratio (DAR value) of ADC-01 was calculated to be 7.98. According to the distribution in which the light chain was conjugated with 0 to 1 toxin molecule and the heavy chain was conjugated with 0 to 3 toxin molecules, n can be 1, 2, 3, 4, 5, 6, 7, or 8.

The target product was obtained using different drug linker conjugates by the same antibody, method, and reaction conditions as described above, and the DAR value was measured by mass spectrometry.

| No. | ADC No. | Structure | DAR value |
|---|---|---|---|
| Ic | ADC-02 | 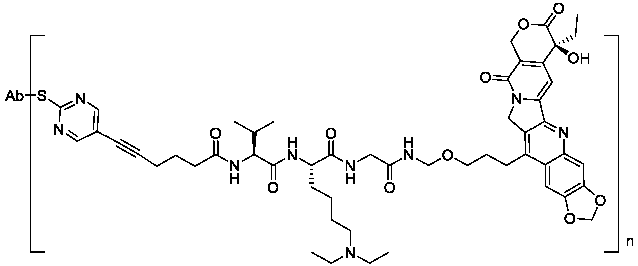 | 7.7 |

### Test example 1: Efficacy assay of ADC on NCI-HT29 xenograft

### 1. Experimental materials

Test compounds: ADC-01 and ADC-02
Experimental cells: NCI-HT29 cells, purchased from ATCC;
Experimental animals: Balb/c nu nude mice, female, 5 to 6 weeks old, purchased from Vital River Laboratory Animal Technology Co., Ltd.

### 2. Experimental scheme

### 2.1. Cell treatment

NCI-HT29 cells were cultured in a 15 cm diameter petri dish with 1640 culture medium containing 10% FBS, digested with trypsin-EDTA when the confluency reached about 80 to 90%, washed twice with PBS, then centrifuged and resuspended in pre-cooled in PBS, counted by a cell counter, and diluted with PBS to a cell concentration of 5 × 10⁷ /mL.

### 2.2. Tumor cell transplantation

After adapting to the laboratory environment for 2 to 5 days, Balb/c nu mice were subcutaneously inoculated with NCI-HT29 cells in the right rib, with an inoculation amount of 5 × 10⁶ cells/mouse and an inoculation volume of 0.2 mL (containing 50% Matrigel). The experiment was carried out when the tumor grew to about 250 mm³.

### 2.3. Administration of animals and detection

The grouped tumor-bearing nude mice were administered according to the following regimen:

| No. | Group | Number of animals | Dose | Route and cycle of administration |
|---|---|---|---|---|
| 1 | ADC-01 | 5 | 3 mg/kg | i.v., QW × 3 |
| 2 | ADC-02 | 5 | 3 mg/kg | i.v., QW × 3 |

After the end of the administration cycle, the mice were continually observed for 1 to 2 weeks. After the last measurement of tumor volume, the tumor was isolated, accurately weighed, and photographed for recording.

### 2.4. Determination of tumor volume and body weight:

Tumor volume and body weight were measured twice a week, and TGI% was calculated.

Tumor volume (V) was calculated according to the formula: V = 1/2 × L_{long} × Lₛₕₒᵣₜ².

### 3. Experimental results

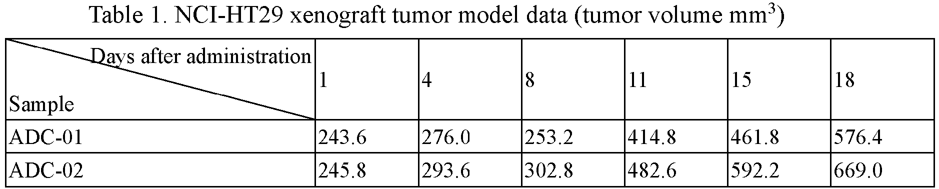

Conclusion: As shown in Table 1, the ADCs of the present application all showed inhibitory effects on tumor growth, especially ADC-01 and ADC-02 had strong tumor inhibitory effects. There was no significant weight loss and no significant drug toxicity in each group of animals during the administration period.

The present application is not limited to the foregoing specific embodiments. The present application extends to any new feature or any new combination disclosed in this specification, as well as to the steps of any new method or process or any new combination disclosed.

## Claims

1. A preparation method for a compound of formula I or a salt thereof, comprising the following steps:
c) reacting a compound of formula 6 or a salt thereof with a compound of formula 7 or a salt thereof to obtain a compound of formula 8 or a salt thereof, 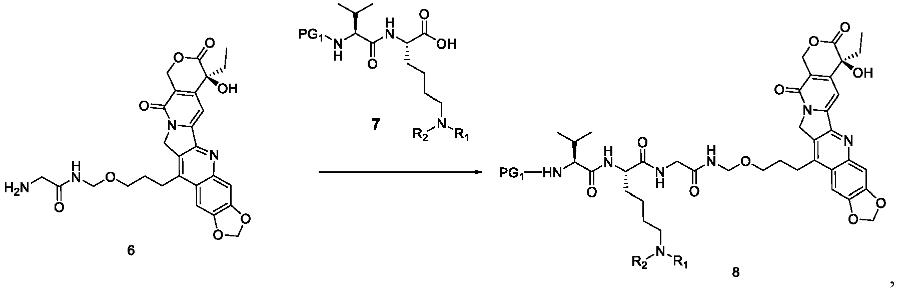
d) reacting the compound of formula 8 or the salt thereof to obtain a compound of formula 9 or a salt thereof, 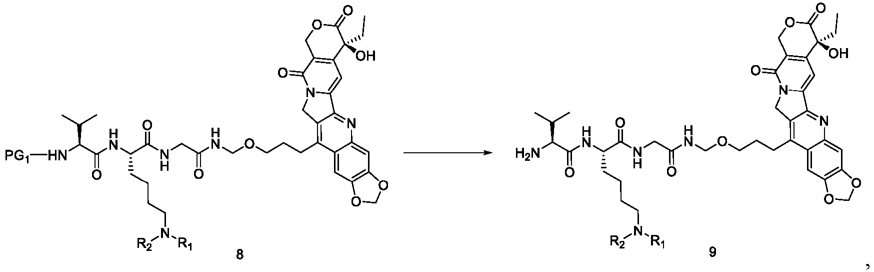
e) reacting the compound of formula 9 or the salt thereof with a compound of formula 10 to obtain a compound of formula I or a salt thereof, 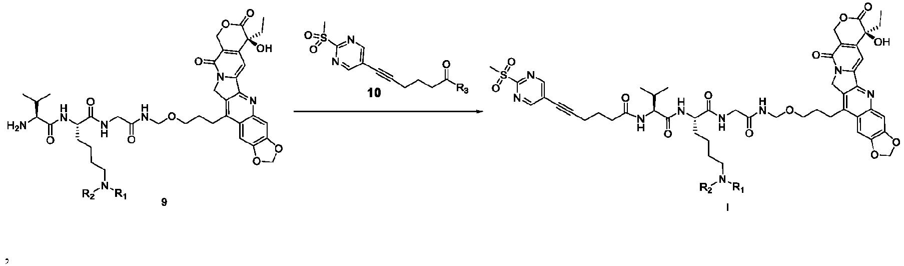 wherein
R₁ and R₂ are each independently selected from C₁₋₄ alkyl;
R₃ is selected from hydroxyl and halogen;
PG₁ is selected from an amino protecting group.

2. The preparation method for the compound of formula **I** or the salt thereof according to claim 1, wherein the preparation method satisfies one or more of the following conditions:
(1) the R₁ and R₂ are each independently selected from methyl, ethyl, and propyl, preferably R₁ and R₂ are the same; preferably, both R₁ and R₂ are propyl;
(2) the R₃ is selected from hydroxyl, fluorine, chlorine, bromine, and iodine; preferably, the R₃ is selected from hydroxyl and chlorine;
(3) the PG₁ is selected from an alkoxycarbonyl protecting group; preferably benzyloxycarbonyl (Cbz), *tert*-butoxycarbonyl (Boc), 9-fluorenylmethoxycarbonyl (Fmoc), allyloxycarbonyl (Alloc), (trimethylsilyl)ethoxycarbonyl (Teoc), methoxycarbonyl, or ethoxycarbonyl; more preferably 9-fluorenylmethoxycarbonyl (Fmoc);
(4) in step c), the compound of formula 6 or the salt thereof is reacted with the compound of formula 7 or the salt thereof in the presence of a condensing agent to obtain the compound of formula 8 or the salt thereof;
further, the condensing agent is selected from DMTMM, HATU, HBTU, HOBt/EDCI, and T₃P; preferably DMTMM or HOBt/EDCI;
further, the molar ratio of the compound of formula 6 or the salt thereof to the condensing agent is selected from 1:0.7 to 1:5; preferably 1:0.7 to 1:2;
(5) in step c), the molar ratio of the compound of formula 6 or the salt thereof to the compound of formula 7 or the salt thereof is selected from 1:0.7 to 1:3; preferably 1:0.7 to 1:2;
(6) in step c), reaction solvent is selected from one of DMF, DMAc, NMP, tetrahydrofuran, 1,4-dioxane, acetonitrile, and dichloromethane, or any combination thereof; preferably one of DMF, dichloromethane, and tetrahydrofuran, or any combination thereof;
(7) in step c), the mass/volume ratio (g/mL) of the compound of formula 6 or the salt thereof to the selected reaction solvent is selected from 1:5 to 1:50;
(8) in step c), reaction temperature is selected from -20 to 50°C; preferably -5 to 25°C;
(9) in step d), the protecting group PG₁ on the amino group is removed from the compound of formula 8 or the salt thereof to obtain the compound of formula 9 or the salt thereof;
preferably, when the PG₁ is 9-fluorenylmethoxycarbonyl (Fmoc), the protecting group PG₁ is removed from the compound of formula 8 or the salt thereof in the presence of a base;
further, the base is selected from piperidine, diethylamine, morpholine, diisopropylamine, DBU, triethylamine, and *N,N-*diisopropylethylamine; for example, diethylamine or *N,N-*diisopropylethylamine; preferably piperidine, diethylamine, or morpholine;
further, the volume ratio of the selected base to a selected reaction solvent is selected from 1:5 to 1:50;
further, the reaction solvent is selected from one of DMF, DMAc, dichloromethane, tetrahydrofuran, 1,4-dioxane, acetonitrile, methanol, and ethanol, or any combination thereof;
further, the mass/volume ratio (g/mL) of the compound of formula 8 or the salt thereof to the selected reaction solvent is selected from 1:5 to 1:50;
further, reaction temperature is selected from -10 to 50°C; preferably 0 to 30°C;
(10) in step e), when the R₃ is hydroxyl, the compound of formula 9 or the salt thereof is reacted with the compound of formula 10 in the presence of a condensing agent to obtain the compound of formula I;
further, the condensing agent is selected from DMTMM, HATU, HBTU, HOBt/EDCI, and T₃P; preferably DMTMM or HOBt/EDCI;
further, the molar ratio of the compound of formula 9 or the salt thereof to the condensing agent is selected from 1:1 to 1:5; preferably 1:1 to 1:2;
further, the molar ratio of the compound of formula 9 or the salt thereof to the compound of formula 10 is selected from 1:0.8 to 1:2.0;
further, reaction solvent is selected from one of DMF, tetrahydrofuran, and dichloromethane, or any combination thereof;
further, the mass/volume ratio (g/mL) of the compound of formula 9 or the salt thereof to the selected reaction solvent is selected from 1:5 to 1:50;
further, reaction temperature is selected from -20 to 50°C; preferably 0 to 30°C;
(11) in step e), when the R₃ is selected from halogen (*e*.*g*., chlorine, fluorine, or bromine), the compound of formula 9 or the salt thereof is reacted with the compound of formula 10 in the presence of a base to obtain the compound of formula I or the salt thereof;
further, the base is selected from triethylamine, *N,N-*diisopropylethylamine, DBU, *N-*methylpiperidine, and *N*-methylmorpholine;
further, the molar ratio of the compound of formula 9 or the salt thereof to the base described in the reaction is selected from 1:1 to 1:5;
further, the molar ratio of the compound of formula 9 or the salt thereof to the compound of formula 10 is selected from 1:0.8 to 1:2.0; preferably 1:1 to 1:1.5;
further, reaction solvent is selected from one of DMF, tetrahydrofuran, and dichloromethane, or any combination thereof;
further, the mass/volume ratio (g/mL) of the compound of formula 9 or the salt thereof to the selected reaction solvent is selected from 1:5 to 1:50;
further, reaction temperature is selected from -20 to 50°C; preferably 0 to 30°C.

3. The preparation method for the compound of formula **I** or the salt thereof according to claim 1, wherein the preparation method further comprises the following steps:
a) reacting a compound of formula 3 or a salt thereof with a compound of formula 4 to obtain a compound of formula 5 or a salt thereof, 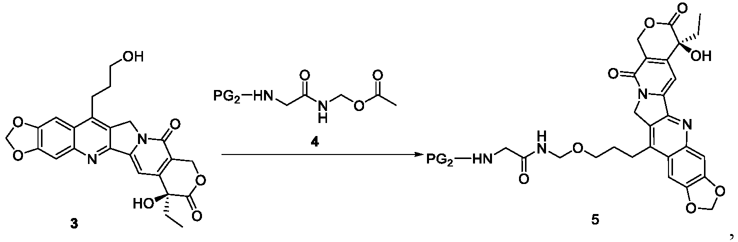
b) reacting the compound of formula 5 or the salt thereof to obtain the compound of formula 6 or the salt thereof, 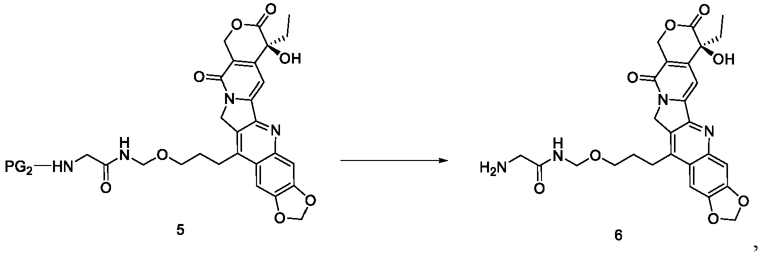 wherein PG₂ is selected from an amino protecting group.

4. The preparation method for the compound of formula I or the salt thereof according to claim 3, wherein the preparation method satisfies one or more of the following conditions:
(1) the PG₂ is selected from an alkoxycarbonyl protecting group; preferably benzyloxycarbonyl (Cbz), *tert*-butoxycarbonyl (Boc), 9-fluorenylmethoxycarbonyl (Fmoc), allyloxycarbonyl (Alloc), (trimethylsilyl)ethoxycarbonyl (Teoc), methoxycarbonyl, or ethoxycarbonyl; more preferably 9-fluorenylmethoxycarbonyl (Fmoc);
(2) in step a), the reaction is carried out in the presence of an acid; further, the acid is selected from hydrogen chloride, hydrobromic acid, sulfuric acid, formic acid, acetic acid, trifluoroacetic acid, *p*-toluenesulfonic acid, pyridinium *p*-toluenesulfonate, zinc acetate, aluminum trichloride (AlCl₃), ferric chloride (FeCl₃), and boron trifluoride diethyl etherate (BF₃·Et₂O); for example, hydrogen chloride or boron trifluoride diethyl etherate; preferably hydrogen chloride, *p*-toluenesulfonic acid, or zinc acetate; further, in step a), the molar ratio of the compound of formula 3 or the salt thereof to the acid selected for the reaction is selected from 1:0.2 to 1:2;
or, in step a), the reaction is carried out in the presence of a base; further, the base is selected from sodium hydroxide, potassium *tert*-butoxide, potassium carbonate, triethylamine (Et₃N), *N,N*-diisopropylethylamine (DIPEA), and pyridine; preferably potassium *tert*-butoxide or sodium hydroxide; further, in step a), the molar ratio of the compound of formula 3 or the salt thereof to the base selected for the reaction is selected from 1:0.5 to 1:3;
(3) in step a), the molar ratio of the compound of formula 3 or the salt thereof to the compound of formula 4 is selected from 1:1 to 1:10; preferably 1:2 to 1:6;
(4) in step a), reaction solvent is selected from an aprotic solvent; preferably an ether, haloalkane, ketone, nitrile, amide, or sulfone solvent; more preferably diethyl ether, diisopropyl ether, methyl *tert*-butyl ether, ethylene glycol diethyl ether, tetrahydrofuran, 1,4-dioxane, dichloromethane, 1,2-dichloroethane, acetone, *N*,*N*-dimethylformamide, *N,N-*dimethylacetamide, or dimethyl sulfoxide; further preferably diisopropyl ether, tetrahydrofuran, 1,4-dioxane, or *N,N-*dimethylformamide;
further, in step a), the mass/volume ratio (g/mL) of the compound of formula 3 or the salt thereof to the selected solvent is selected from 1:5 to 1:50; preferably 1:8 to 1:30;
(5) in step a), reaction temperature is selected from -10 to 110°C; preferably 0 to 60°C;
(6) in step b), the protecting group PG₂ on the amino group is removed from the compound of formula 5 or the salt thereof to obtain the compound of formula 6 or the salt thereof;
preferably, when the PG₂ is 9-fluorenylmethoxycarbonyl (Fmoc), the protecting group PG₂ is removed from the compound of formula 5 or the salt thereof in the presence of a base;
further, the base is selected from piperidine, diethylamine, morpholine, diisopropylamine, DBU, triethylamine, and *N,N-*diisopropylethylamine; preferably piperidine, diethylamine, or morpholine;
further, the molar ratio of the compound of formula 5 or the salt thereof to the selected base is selected from 1:0.2 to 1:40; preferably 1:1 to 1: 10;
(7) in step b), when the PG₂ is 9-fluorenylmethoxycarbonyl (Fmoc), reaction solvent is selected from one of DMF, DMAc, dichloromethane, tetrahydrofuran, 1,4-dioxane, acetonitrile, methanol, and ethanol, or any combination thereof; preferably one of DMF and dichloromethane, or any combination thereof;
(8) in step b), when the PG₂ is 9-fluorenylmethoxycarbonyl (Fmoc), the mass/volume ratio (g/mL) of the compound of formula 5 or the salt thereof to the selected reaction solvent is selected from 1:5 to 1:50; preferably 1:10 to 1:30;
(9) in step b), when the PG₂ is 9-fluorenylmethoxycarbonyl (Fmoc), reaction temperature is selected from 0 to 50°C; preferably 10 to 30°C.

5. The preparation method for the compound of formula **I** or the salt thereof according to claim 3, wherein the preparation method further comprises the following steps:
h) reacting a compound of formula 1 or a salt thereof with a compound of formula 2 to obtain the compound of formula 3 or the salt thereof, 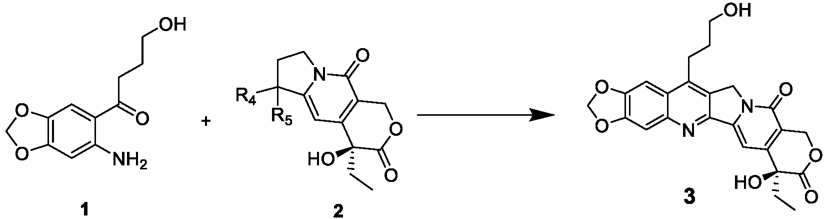 wherein
R₄ and R₅ are each independently selected from -OC₁₋₄ alkyl, -SC₁₋₄ alkyl, and -NHC₁₋₄ alkyl;
or, R₄ and R₅ together with the carbon atom to which they are attached form a carbonyl group;
or, R₄ and R₅ together with the carbon atom to which they are attached form 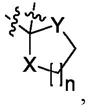 wherein X and Y are each independently selected from O, S, NH, and NC₁₋₄ alkyl, and n is selected from 1, 2, and 3.

6. The preparation method for the compound of formula **I** or the salt thereof according to claim 5, wherein the preparation method satisfies one or more of the following conditions:
(1) the R₄ and R₅ are each independently selected from methoxy, ethoxy, propoxy, methylthio, ethylthio, propylthio, methylamino, ethylamino, and propylamino; or, R₄ and R₅ together with the carbon atom to which they are attached form a carbonyl group; or, R₄ and R₅ together with the carbon atom to which they are attached form 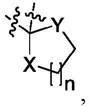 wherein X and Y are each independently selected from O, S, NH, and NC₁₋₄ alkyl, and n is selected from 1, 2, and 3;
further, the R₄ and R₅ together with the carbon atom to which they are attached form a carbonyl group; or, R₄ and R₅ together with the carbon atom to which they are attached form 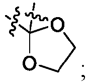
(2) in step h), the reaction is carried out in the presence of an acid;
further, the acid is selected from one of *p*-toluenesulfonic acid monohydrate, pyridinium *p*-toluenesulfonate, trifluoroacetic acid, and acetic acid, or any combination thereof; preferably *p*-toluenesulfonic acid monohydrate;
further, the molar ratio of the compound of formula 2 to the selected acid is selected from 1:0.2 to 1:2; preferably 1:0.2 to 1:0.4;
further, the compound of formula 1 is added to the reaction system in batches; preferably, based on the total amount added, in the presence of the acid, 25 to 35 mol% of the compound of formula 1 or the salt thereof is reacted with the compound of formula 2 at 100 to 120°C, and the remaining compound of formula 1 is added to the reaction system in 2 to 5 batches; more preferably, based on the total addition amount, in the presence of the acid, 25 to 35 mol% of the compound of formula 1 or the salt thereof is reacted with the compound of formula 2 at 100 to 120°C for 20 to 40 minutes, and the remaining compound of formula 1 is added to the reaction system in 2 to 5 batches with an interval of 20 to 40 minutes, and after the addition is completed, the reaction is continued for 30 to 90 minutes;
(3) in step h), reaction solvent is selected from one of tetrahydrofuran, 1,4-dioxane, toluene, *o*-methylphenol, *N*,*N*-dimethylformamide, *N,N-*dimethylacetamide, and *N-*methylpyrrolidone, or any combination thereof; preferably one of toluene, *o*-methylphenol, and N-methylpyrrolidone, or any combination thereof; more preferably *N*-methylpyrrolidone;
(4) in step h), the mass/volume ratio (g/mL) of the compound of formula 2 to reaction solvent is selected from 1: 1 to 1:60;
(5) in step h), the molar ratio of the compound of formula 1 or the salt thereof to the compound of formula 2 is selected from 3:1 to 1:1; preferably 2:1 to 1:1;
(6) in step h), reaction temperature is selected from 70 to 130°C; preferably 80 to 120°C; more preferably 100 to 120°C.

7. The preparation method for the compound of formula **I** or the salt thereof according to claim 5, wherein the preparation method further comprises method I or method II as follows:
method I comprises the following steps:
step i-1) reacting a compound of formula 1-A1 with a compound of formula 1-A2 to obtain a compound of formula 1-A3;
step i-2) reacting the compound of formula 1-A3 to obtain the compound of formula 1 or the salt thereof; 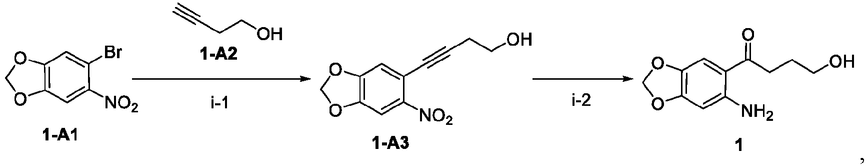
method II comprises the following steps:
step j-1) reacting a compound of formula 1-B1 with a compound of formula 1-B2 to obtain a compound of formula 1-B3;
step j-2) reacting the compound of formula 1-B3 to obtain a compound of formula 1-B4;
step j-3) reacting the compound of formula 1-B4 to obtain a compound of formula 1-B5 or a salt thereof;
step j-4) reacting the compound of formula 1-B5 or the salt thereof to obtain the compound of formula 1 or the salt thereof; 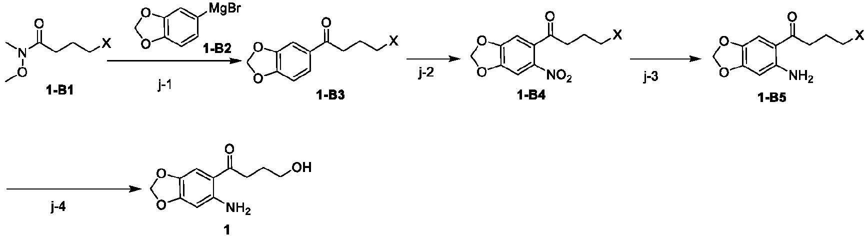
wherein X is selected from halogen.

8. The preparation method for the compound of formula **I** or the salt thereof according to claim 7, wherein the preparation method satisfies one or more of the following conditions:
(1) the X is selected from fluorine, chlorine, bromine, and iodine; preferably chlorine;
(2) in step i-1), the compound of formula 1-A1 is reacted with the compound of formula 1-A2 in the presence of a palladium catalyst, a copper catalyst, and a base to obtain the compound of formula 1-A3;
further, the palladium catalyst is selected from Pd(PPh₃)₄, Pd(OAc)₂, Pd(PPh₃)₂Cl₂, and Pd₂(dba)₃, preferably Pd(PPh₃)₂Cl₂; further, the molar ratio of the palladium catalyst to the compound of formula 1-A1 is selected from 0.01:1 to 0.2:1; preferably 0.01:1 to 0.10:1; more preferably 0.01:1 to 0.03:1;
further, the copper catalyst is selected from CuI, CuBr, and CuCl, preferably CuI; further, the molar ratio of the copper catalyst in the reaction to the compound of formula 1-A1 is selected from 0.01:1 to 0.2:1; preferably 0.02:1 to 0.10:1; more preferably 0.02:1 to 0.05:1;
further, the base is selected from triethylamine, *N,N-*diisopropylethylamine, DBU, potassium carbonate, and cesium carbonate, preferably triethylamine or *N,N-*diisopropylethylamine; further, in the reaction, the molar ratio of the base to the compound of formula 1-A1 is selected from 10:1 to 1:1; preferably 5:1 to 2:1;
(3) in step i-1), the molar ratio of the compound of formula 1-A1 to the compound of formula 1-A2 is selected from 1:1 to 1:5; preferably 1:1 to 1:3; more preferably 1:1 to 1:2;
(4) in step i-1), reaction solvent is selected from one of tetrahydrofuran, 1,4-dioxane, toluene, *N*-methylpyrrolidone, *N*,*N*-dimethylformamide, and *N*,*N*-dimethylacetamide, or any combination thereof; for example, the reaction solvent is selected from one of tetrahydrofuran, 1,4-dioxane, toluene, *N,N-*dimethylformamide, and *N,N*-dimethylacetamide, or any combination thereof; for another example, tetrahydrofuran, *N*-methylpyrrolidone, or *N,N-*dimethylformamide; preferably tetrahydrofuran or 1,4-dioxane;
(5) in step i-1), the mass/volume ratio (g/mL) of the compound of formula 1-A1 to reaction solvent is selected from 1:5 to 1:20; preferably 1:10 to 1:20;
(6) in step i-1), reaction temperature is selected from 40 to 80°C; preferably 40 to 60°C;
(7) in step i-2), the compound of formula 1-A3 is reacted in the presence of Sn and Na₂S to obtain the compound of formula 1 or the salt thereof; Na₂S is added to the system in the form of Na₂S·2H₂O and/or Na₂S·9H₂O;
further, the molar ratio of the Sn to the compound of formula 1-A3 is selected from 5:1 to 1:1; preferably 3:1 to 1:1;
further, the molar ratio of the Na₂S to the compound of formula 1-A3 is selected from 0.1:1 to 2:1; preferably 0.2:1 to 1:1;
(8) in step i-2), reaction solvent is selected from one of tetrahydrofuran, acetone, acetonitrile, 1,4-dioxane, methanol, ethanol, propanol, and water, or any combination thereof; preferably one of tetrahydrofuran, ethanol, and methanol, or any combination thereof; more preferably a combination of ethanol and water, wherein the volume ratio of ethanol to water is selected from 20:1 to 1:1, preferably 10:1 to 5:1;
(9) in step i-2), reaction temperature is selected from 40 to 100°C; for example, 50 to 80°C; preferably 60 to 90°C;
(10) in step j-1), the compound of formula 1-B1 undergoes a Grignard reaction with the compound of formula 1-B2 to obtain the compound of formula 1-B3;
(11) in step j-1), the molar ratio of the compound of formula 1-B1 to the compound of formula 1-B2 is selected from 1:0.8 to 1:2; preferably 1:1 to 1:1.5;
(12) in step j-1), reaction solvent is selected from one of tetrahydrofuran, 2-methyltetrahydrofuran, diisopropyl ether, and diethyl ether, or any combination thereof; preferably tetrahydrofuran or diisopropyl ether;
(13) in step j-1), reaction temperature is selected from -20 to 60°C; preferably -20 to 50°C; more preferably 0 to 30°C;
(14) in step j-2), the compound of formula 1-B3 undergoes a nitration reaction to obtain the compound of formula 1-B4;
further, nitration system of the reaction is selected from concentrated nitric acid, concentrated nitric acid/acetic acid, and concentrated sulfuric acid/potassium nitrate;
further, the mass/volume ratio (g/mL) of the compound of formula 1-B3 to the nitration system is selected from 1:4 to 1:20; preferably 1:6 to 1:15;
(15) in step j-2), reaction temperature is selected from -20 to 30°C; preferably -20 to 0°C;
(16) in step j-3), the nitro group of the compound of formula 1-B4 is reduced to an amino group in the presence of a reducing agent to obtain the compound of formula 1-B5 or the salt thereof;
further, the reducing agent is selected from hydrogen, TiCl₃, SnCl₂, sodium dithionite, zinc powder, and iron powder; preferably hydrogen, SnCl₂, or sodium dithionite;
further, the reaction is carried out under hydrogen atmosphere with the addition of a transition metal catalyst, and the transition metal catalyst is preferably selected from platinum dioxide, palladium on carbon, rhodium on carbon, and Raney nickel; further, when the reaction is carried out under a hydrogen-platinum dioxide system, the mass ratio of the compound of formula 1-B4 to the platinum dioxide is selected from 1:0.01 to 1:0.4; preferably 1:0.01 to 1:0.1; when the reaction is carried out under the hydrogen-platinum dioxide system, hydrogen pressure is selected from 1 to 10 atmospheres, preferably 1 to 5 atmospheres; reaction temperature is selected from -10 to 100°C; preferably -10 to 80°C; reaction solvent is selected from one of methanol, ethanol, ethyl acetate, tetrahydrofuran, and 1,4-dioxane, or any combination thereof; preferably ethyl acetate or tetrahydrofuran;
(17) in step j-4), the reaction is carried out in the presence of a base; further, the base is selected from potassium carbonate, potassium hydroxide, sodium hydroxide, cesium carbonate, and sodium carbonate; for example, the base is selected from potassium carbonate, sodium hydroxide, cesium carbonate, and sodium carbonate; for another example, potassium carbonate or potassium hydroxide; preferably potassium carbonate or sodium carbonate; further, the molar ratio of the compound of formula 1-B5 to the base is selected from 1:1 to 1:5; preferably 1:1 to 1:3;
(18) in step j-4), reaction solvent is selected from one of water, tetrahydrofuran, 1,4-dioxane, acetonitrile, hexamethylphosphoramide, *N,N-*dimethylformamide, *N,N-*dimethylacetamide, *N*-methylpyrrolidone, and dimethyl sulfoxide, or any combination thereof; preferably one of water, acetonitrile, tetrahydrofuran, dimethyl sulfoxide, and hexamethylphosphoramide, or any combination thereof; further, in step j-4), the reaction solvent is selected from a combination of acetonitrile and water, wherein the volume ratio of acetonitrile to water is selected from 1:5 to 10:1; preferably 1:2 to 5:1;
(19) in step j-4), the mass/volume ratio (g/mL) of the compound of formula 1-B5 or the salt thereof to reaction solvent is selected from 1:5 to 1:20; preferably 1:5 to 1:10;
(20) in step j-4), reaction temperature is selected from 20 to 100°C; preferably 40 to 80°C.

9. The preparation method for the compound of formula **I** or the salt thereof according to claim 1, wherein the preparation method further comprises method I or method II as follows:
method I comprises the following steps:
f-1) reacting a compound of formula 7-A1 with a compound of formula 7-A2 or a salt thereof to obtain a compound of formula 7-A3, 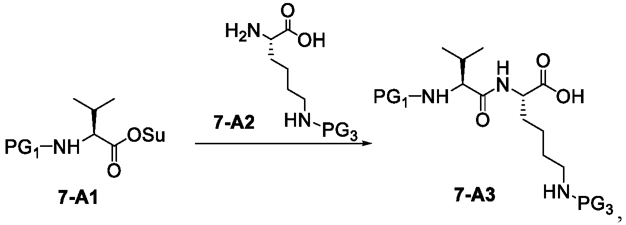
f-2) reacting the compound of formula 7-A3 to obtain a compound of formula 7-A4 or a salt thereof, 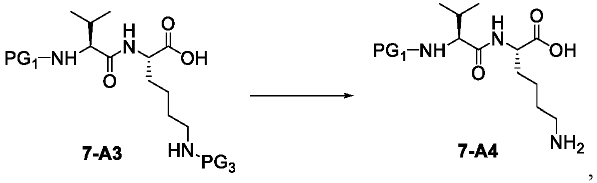
f-3) reacting the compound of formula 7-A4 or the salt thereof to obtain the compound of formula 7 or the salt thereof, 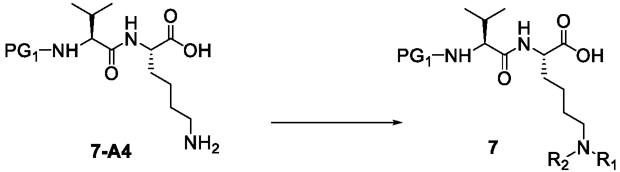 wherein
R₁ and R₂ are each independently selected from C₁₋₄ alkyl;
PG₁ and PG₃ are each independently selected from an amino protecting group, and PG₁ and PG₃ are different;
method II comprises the following steps:
g-1) reacting a compound of formula 7-B1 or a salt thereof to obtain a compound of formula 7-B2 or a salt thereof, 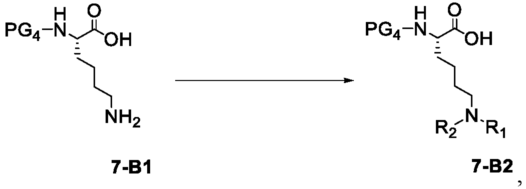
g-2) reacting the compound of formula 7-B2 or the salt thereof to obtain a compound of formula 7-B3 or a salt thereof, 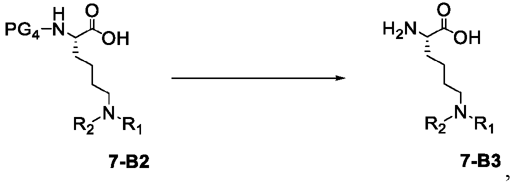
g-3) reacting the compound of formula 7-B3 or the salt thereof with the compound of formula 7-A1 to obtain the compound of formula 7 or the salt thereof, 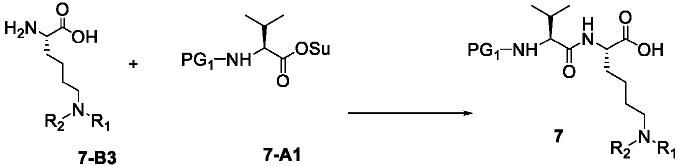 wherein
R₁ and R₂ are each independently selected from C₁₋₄ alkyl;
PG₁ and PG₄ are each independently selected from an amino protecting group.

10. The preparation method for the compound of formula **I** or the salt thereof according to claim 9, wherein the preparation method satisfies one or more of the following conditions:
(1) the R₁ and R₂ are selected from methyl, ethyl, and propyl, preferably R₁ and R₂ are the same; preferably, both R₁ and R₂ are propyl;
(2) the PG₁ is selected from an alkoxycarbonyl protecting group; preferably benzyloxycarbonyl (Cbz), *tert*-butoxycarbonyl (Boc), 9-fluorenylmethoxycarbonyl (Fmoc), allyloxycarbonyl (Alloc), (trimethylsilyl)ethoxycarbonyl (Teoc), methoxycarbonyl, or ethoxycarbonyl; more preferably 9-fluorenylmethoxycarbonyl (Fmoc);
(3) the PG₃ is selected from an alkoxycarbonyl protecting group; preferably benzyloxycarbonyl (Cbz), *tert*-butoxycarbonyl (Boc), 9-fluorenylmethoxycarbonyl (Fmoc), allyloxycarbonyl (Alloc), (trimethylsilyl)ethoxycarbonyl (Teoc), methoxycarbonyl, or ethoxycarbonyl; more preferably *tert*-butoxycarbonyl (Boc);
(4) the PG₄ is selected from an alkoxycarbonyl protecting group; preferably benzyloxycarbonyl (Cbz), *tert*-butoxycarbonyl (Boc), 9-fluorenylmethoxycarbonyl (Fmoc), allyloxycarbonyl (Alloc), (trimethylsilyl)ethoxycarbonyl (Teoc), methoxycarbonyl, or ethoxycarbonyl; more preferably 9-fluorenylmethoxycarbonyl (Fmoc);
(5) in step f-1), the reaction is carried out in the presence of a base; further, the base is selected from sodium bicarbonate, sodium carbonate, potassium carbonate, cesium carbonate, and potassium bicarbonate; for example, sodium bicarbonate or sodium carbonate; preferably sodium bicarbonate, potassium bicarbonate, or potassium carbonate;
(6) in step f-1), reaction solvent is selected from one of tetrahydrofuran, acetone, acetonitrile, 1,4-dioxane, *N,N-*dimethylformamide, *N,N*-dimethylacetamide, dimethyl sulfoxide, and water, or any combination thereof; preferably one of tetrahydrofuran, acetone, acetonitrile, and water, or any combination thereof;
(7) in step f-1), the mass/volume ratio (g/mL) of the compound of formula 7-A1 or the salt thereof to reaction solvent is selected from 1:5 to 1:20;
(8) in step f-1), the molar ratio of the compound of formula 7-A1 to the compound of formula 7-A2 or the salt thereof is selected from 1:1 to 1:3;
(9) in step f-1), reaction temperature is selected from 0 to 50°C; preferably 0 to 30°C;
(10) in step f-2), the protecting group PG₃ on the amino group is removed from the compound of formula 7-A3 to obtain the compound of formula 7-A4;
preferably, when the PG₃ is *tert*-butoxycarbonyl (Boc), the protecting group PG₃ is removed from the compound of formula 7-A3 in the presence of an acid;
further, the acid is selected from trifluoroacetic acid, hydrogen chloride, hydrobromic acid, and sulfuric acid; preferably hydrogen chloride or trifluoroacetic acid;
further, when the PG₃ is *tert*-butoxycarbonyl (Boc), the molar ratio of the compound of formula 7-A3 to the selected acid is selected from 1:5 to 1:50; preferably 1:10 to 1:30;
(11) in step f-2), when the PG₃ is *tert*-butoxycarbonyl (Boc), reaction solvent is selected from one of tetrahydrofuran, 1,4-dioxane, ethyl acetate, and methyl *tert*-butyl ether, or any combination thereof; preferably tetrahydrofuran or 1,4-dioxane;
(12) in step f-2), when the PG₃ is *tert*-butoxycarbonyl (Boc), the mass/volume ratio (g/mL) of the compound of formula 7-A3 to the selected reaction solvent is selected from 1:4 to 1:20; preferably 1:5 to 1:15;
(13) in step f-2), when the PG₃ is *tert*-butoxycarbonyl (Boc), reaction temperature is selected from 0 to 50°C; preferably 0 to 30°C;
(14) in step f-3), the compound of formula 7-A4 or the salt thereof undergoes a reductive amination reaction with an aldehyde in the presence of a reducing agent and an acid to obtain the compound of formula 7 or the salt thereof;
further, the reducing agent is selected from sodium borohydride, sodium cyanoborohydride, sodium triacetoxyborohydride, and Pd/C-H₂; preferably sodium cyanoborohydride and/or sodium triacetoxyborohydride;
further, the molar ratio of the compound of formula 7-A4 or the salt thereof to the selected reducing agent is selected from 1:2 to 1:10; preferably 1:3 to 1:8; further, the temperature at which the reducing agent is added is selected from -20 to 30°C; preferably 0°C;
further, the acid is selected from acetic acid, formic acid, propionic acid, trifluoroacetic acid, and hydrochloric acid; preferably acetic acid or propionic acid;
further, the molar ratio of the compound of formula 7-A4 or the salt thereof to the aldehyde is selected from 1:2 to 1:10; preferably 1:4 to 1:8;
(15) in step f-3), reaction solvent is selected from one of methanol, ethanol, propanol, tetrahydrofuran, 1,4-dioxane, and ethyl acetate, or any combination thereof; preferably methanol or ethanol;
(16) in step f-3), the mass/volume ratio (g/mL) of the compound of formula 7-A4 or the salt thereof to the reaction solvent is selected from 1:5 to 1:20; preferably 1:6 to 1: 15;
(17) in step f-3), reaction temperature is selected from -20 to 50°C; preferably 0 to 30°C;
(18) in step g-1), the compound of formula 7-B1 or the salt thereof undergoes a reductive amination reaction with an aldehyde in the presence of a reducing agent and an acid to obtain the compound of formula 7-B2 or the salt thereof;
further, the reducing agent is selected from sodium borohydride, sodium cyanoborohydride, sodium triacetoxyborohydride, and Pd/C-H₂; preferably sodium cyanoborohydride or sodium triacetoxyborohydride;
further, the molar ratio of the compound of formula 7-B1 or the salt thereof to the selected reducing agent is selected from 1:2 to 1: 10;
further, the temperature at which the reducing agent is added is selected from -20 to 30°C; further, the acid is selected from acetic acid, formic acid, propionic acid, trifluoroacetic acid, and hydrochloric acid; preferably acetic acid or propionic acid;
further, the molar ratio of the compound of formula 7-B1 or the salt thereof to the aldehyde is selected from 1:2 to 1:10;
(19) in step g-1), reaction solvent is selected from one of methanol, ethanol, propanol, tetrahydrofuran, 1,4-dioxane, and ethyl acetate, or any combination thereof; preferably methanol or ethanol;
(20) in step g-1), the mass/volume ratio (g/mL) of the compound of formula 7-B1 or the salt thereof to the reaction solvent is selected from 1:5 to 1:20;
(21) in step g-1), reaction temperature is selected from -20 to 50°C; preferably 0 to 30°C;
(22) in step g-2), the protecting group PG₄ on the amino group is removed from the compound of formula 7-B2 or the salt thereof to obtain the compound of formula 7-B3 or the salt thereof;
preferably, when the PG₄ is 9-fluorenylmethoxycarbonyl (Fmoc), the protecting group PG₄ is removed from the compound of formula 7-B2 or the salt thereof in the presence of a base;
further, when the PG₄ is 9-fluorenylmethoxycarbonyl (Fmoc), the base is selected from piperidine, diethylamine, morpholine, diisopropylamine, DBU, triethylamine, and *N,N-*diisopropylethylamine; preferably piperidine, diethylamine, or morpholine;
further, when the PG₄ is 9-fluorenylmethoxycarbonyl (Fmoc), the mass/volume ratio (g/mL) of the selected base to reaction solvent is selected from 1:5 to 1:50;
further, when the PG₄ is 9-fluorenylmethoxycarbonyl (Fmoc), the reaction solvent is selected from one of DMF, DMAc, dichloromethane, tetrahydrofuran, 1,4-dioxane, acetonitrile, methanol, and ethanol, or any combination thereof; preferably one of DMF, tetrahydrofuran, and dichloromethane, or any combination thereof;
further, when the PG₄ is 9-fluorenylmethoxycarbonyl (Fmoc), the mass/volume ratio (g/mL) of the compound of formula 7-B2 or the salt thereof to the selected reaction solvent is selected from 1:2 to 1:20;
further, when the PG₄ is 9-fluorenylmethoxycarbonyl (Fmoc), reaction temperature is selected from 0 to 50°C; preferably 0 to 30°C;
(23) in step g-3), the reaction is carried out in the presence of a base;
further, the base is selected from sodium bicarbonate, sodium carbonate, potassium carbonate, cesium carbonate, and potassium bicarbonate; preferably sodium bicarbonate, potassium bicarbonate, or potassium carbonate;
(24) in step g-3), reaction solvent is selected from one of tetrahydrofuran, acetone, acetonitrile, 1,4-dioxane, *N,N-*dimethylformamide, *N,N*-dimethylacetamide, dimethyl sulfoxide, and water, or any combination thereof; preferably one of water, tetrahydrofuran, acetone, and acetonitrile, or any combination thereof; more preferably a combination of acetone and water; further, the reaction solvent is selected from a combination of acetone and water, wherein the volume ratio of acetone to water is selected from 1:0.2 to 1:10;
(25) in step g-3), the molar ratio of the compound of formula 7-B3 or the salt thereof to the compound of 7-A1 is selected from 1:1 to 1:5;
(26) in step g-3), reaction temperature is selected from 0 to 50°C.

11. A preparation method for a compound of formula 3 or a salt thereof, comprising the following steps:
h) reacting a compound of formula 1 or a salt thereof with a compound of formula 2 to obtain a compound of formula 3 or the salt thereof, 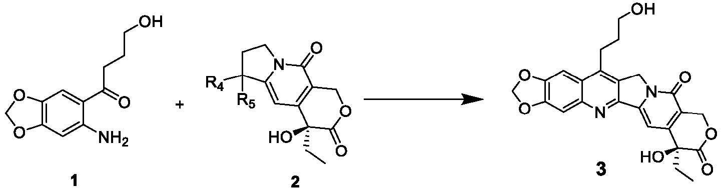 wherein
R₄ and R₅ are each independently selected from -OC₁₋₄ alkyl, -SC₁₋₄ alkyl, and -NHC₁₋₄ alkyl;
or, R₄ and R₅ together with the carbon atom to which they are attached form a carbonyl group;
or, R₄ and R₅ together with the carbon atom to which they are attached form 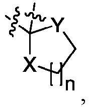 wherein X and Y are each independently selected from O, S, NH, and NC₁₋₄ alkyl, and n is selected from 1, 2, and 3;
further, R₄ and R₅ are independently as defined in claim 6;
further, operations and conditions of step h) are independently as defined in claim 6.

12. A preparation method for a compound of formula 1, which is method I or method II as follows:
method I comprises the following steps:
step i-1) reacting a compound of formula 1-A1 with a compound of formula 1-A2 to obtain a compound of formula 1-A3;
step i-2) reacting the compound of formula 1-A3 to obtain a compound of formula 1 or a salt thereof; 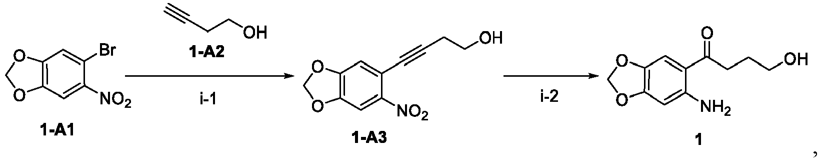
method II comprises the following steps:
step j-1) reacting a compound of formula 1-B1 with a compound of formula 1-B2 to obtain a compound of formula 1-B3;
step j-2) reacting the compound of formula 1-B3 to obtain a compound of formula 1-B4;
step j-3) reacting the compound of formula 1-B4 to obtain a compound of formula 1-B5 or a salt thereof;
step j-4) reacting the compound of formula 1-B5 or the salt thereof to obtain a compound of formula 1 or the salt thereof; 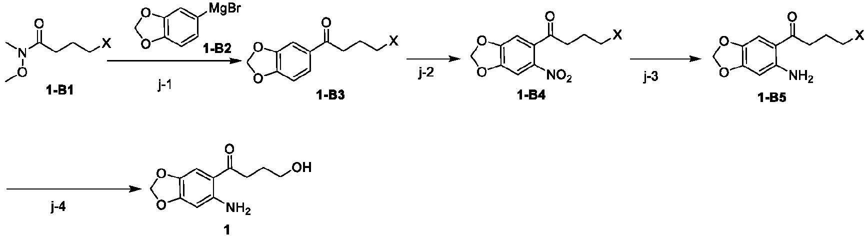
wherein X is selected from halogen;
further, X is independently as defined in claim 8;
further, operations and conditions of step i-1), step i-2), step j-1), step j-2), step j-3), and step j-4) are independently as defined in claim 8.

13. A preparation method for a compound of formula 7 or a salt thereof, which is method I or method II as follows:
method I comprises the following steps:
f-1) reacting a compound of formula 7-A1 with a compound of formula 7-A2 or a salt thereof to obtain a compound of formula 7-A3, 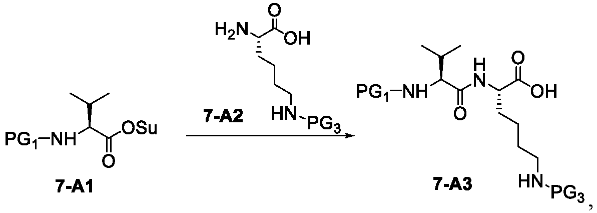
f-2) reacting the compound of formula 7-A3 to obtain a compound of formula 7-A4 or a salt thereof, 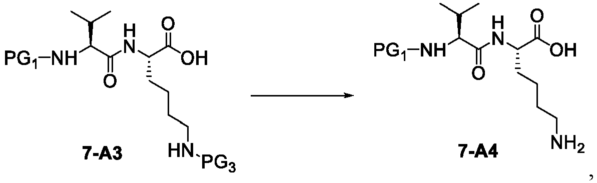
f-3) reacting the compound of formula 7-A4 or the salt thereof to obtain a compound of formula 7 or the salt thereof, 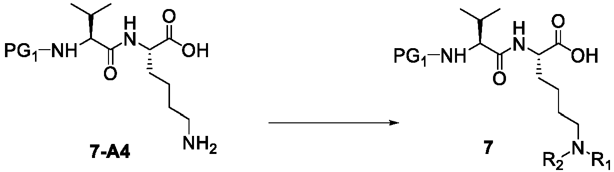 wherein
R₁ and R₂ are each independently selected from C₁₋₄ alkyl;
PG₁ and PG₃ are each independently selected from an amino protecting group, and PG₁ and PG₃ are different;
method II comprises the following steps:
g-1) reacting a compound of formula 7-B1 or a salt thereof to obtain a compound of formula 7-B2 or a salt thereof, 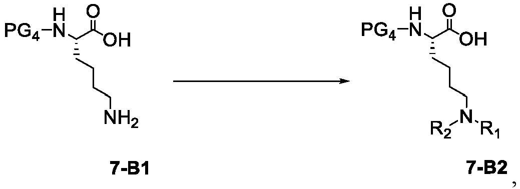
g-2) reacting the compound of formula 7-B2 or the salt thereof to obtain a compound of formula 7-B3 or a salt thereof, 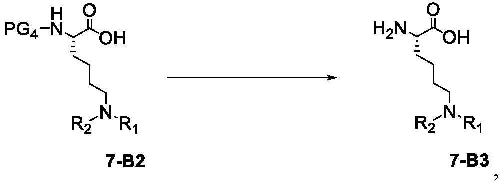
g-3) reacting the compound of formula 7-B3 or the salt thereof with a compound of formula 7-A1 to obtain a compound of formula 7 or the salt thereof, 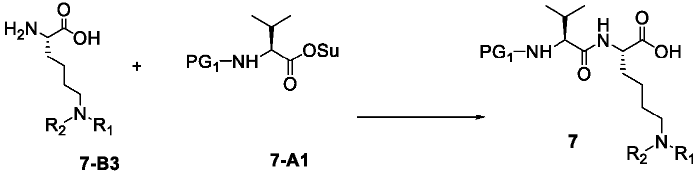 wherein
R₁ and R₂ are each independently selected from C₁₋₄ alkyl;
PG₁ and PG₄ are each independently selected from an amino protecting group;
further, R₁, R₂, PG₁, PG₃, and PG₄ are independently as defined in claim 10;
further, operations and conditions of step f-1), step f-2), step f-3), step g-1), step g-2), and step g-3) are independently as defined in claim 10.

14. A compound of the following formulas or a salt thereof, 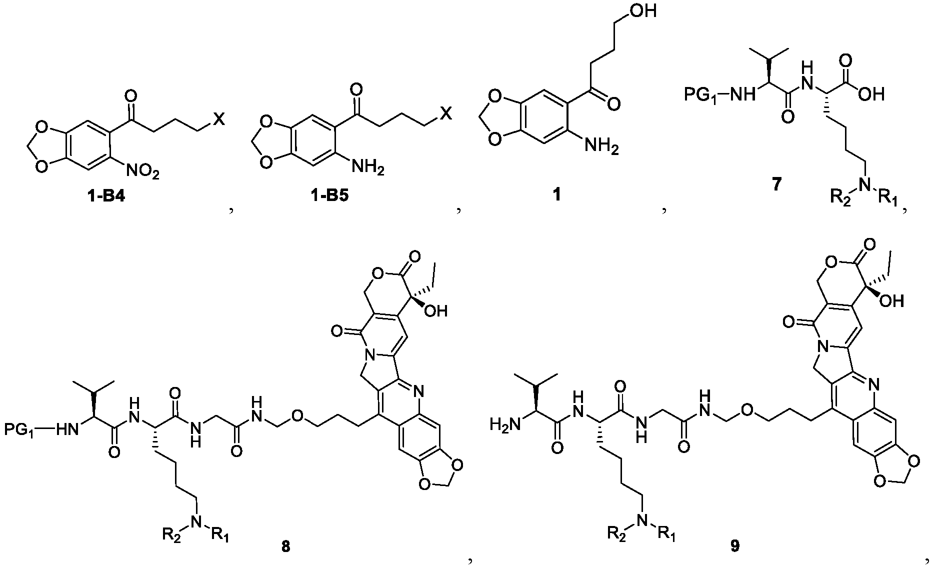 wherein
R₁ and R₂ are each independently selected from C₁₋₄ alkyl;
X is selected from halogen;
PG₁ is selected from an amino protecting group;
the compound of formula 7 is not

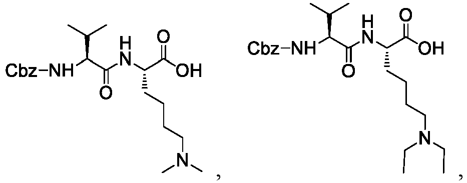

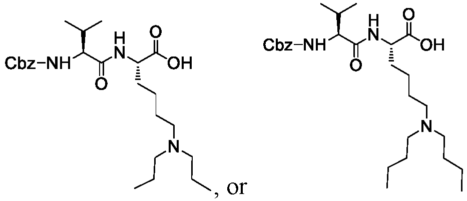

15. The compound of the following formulas or the salt thereof according to claim 14, wherein the compound satisfies one or more of the following conditions:
(1) the R₁ and R₂ are each independently selected from methyl, ethyl, and propyl, preferably R₁ and R₂ are the same; preferably, both R₁ and R₂ are propyl;
(2) the X is selected from fluorine, chlorine, bromine, or iodine; preferably, the X is chlorine;
(3) the PG₁ is selected from an alkoxycarbonyl protecting group; preferably benzyloxycarbonyl (Cbz), tert-butoxycarbonyl (Boc), 9-fluorenylmethoxycarbonyl (Fmoc), allyloxycarbonyl (Alloc), (trimethylsilyl)ethoxycarbonyl (Teoc), methoxycarbonyl, or ethoxycarbonyl; more preferably 9-fluorenylmethoxycarbonyl (Fmoc).

16. The compound of the following formulas or the salt thereof according to claim 15, wherein the compound is selected from:
